# EUROPEAN PATENT SPECIFICATION

(11) **EP 1 545 557 B1**
(45) Date of publication and mention of the grant of the patent: **08.02.2012**
(21) Application number: 03764562.9
(22) Date of filing: 11.07.2003
(51) Int. Cl.: A61K 31/513, A61K 31/7072, A61K 38/21, A61P 31/12

(54) **COMBINATION THERAPIES WITH L-FMAU FOR THE TREATMENT OF HEPATITIS B VIRUS INFECTION**
KOMBINATIONSTHERAPIEN MIT L-FMAU ZUR BEHANDLUNG VON HEPATITIS-B-VIRUS-INFEKTIONEN
POLYTHERAPIES A BASE DE L-FMAU POUR LE TRAITEMENT DE L'INFECTION PAR LE VIRUS DE L'HEPATITE B

(30) Priority: 15.07.2002 US 396117 P
(43) Date of publication of application: 29.06.2005
(73) Proprietor: GILEAD SCIENCES, INC., Foster City, California 94404 (US)
(72) Inventor: FURMAN, Phillip, A., Durham, NC 27705 (US)
(74) Representative: Hallybone, Huw George
(86) International application number: PCT/US2003/021811
(87) International publication number: WO 2004/006843

(56) References cited:
- US-A- 5 145 677
- US-A- 5 486 520
- US-A- 5 703 058
- SEIGNERES B ET AL: "Enhancement of the antiviral effect by the combination of pyrimidine and purine analogs in vitro and in vivo in the DHBV model" ANTIVIRAL RESEARCH, ELSEVIER SCIENCE BV., AMSTERDAM, NL, vol. 53, no. 3, 1 March 2002 (2002-03-01), page A72, XP009115765 ISSN: 0166-3542
- DELANEY WILLIAM 4TH ET AL: "Evolving therapies for the treatment of chronic hepatitis B virus infection." EXPERT OPINION ON INVESTIGATIONAL DRUGS FEB 2002, vol. 11, no. 2, February 2002 (2002-02), pages 169-187, XP002525228 ISSN: 1354-3784
- PICHOUD C ET AL: "Transient inhibition of WHV replication induced by adenovirus mediated delivery of interferon gamma" ANTIVIRAL RESEARCH, ELSEVIER SCIENCE BV., AMSTERDAM, NL, vol. 53, no. 3, 1 March 2002 (2002-03-01), page A72, XP009115770 ISSN: 0166-3542
- JACQUARD ANNE-CAROLE ET AL: "Evaluation of a combination of clevudine and emtricitabine with adenovirus mediated delivery of interferon gamma in the woodchuck model of HBV infection" HEPATOLOGY, WILLIAMS AND WILKINS, BALTIMORE, MD, US, vol. 36, no. 4 Part 2, 1 October 2002 (2002-10-01), page 374A, XP009115673 ISSN: 0270-9139

## Description

### FIELD OF THE INVENTION

The present invention describes uses, compositions and compounds for use in the treatment or prophylaxis of a human infected with hepatitis B virus, as set out in the claims. It relates to the treatment or prophylaxis of a hepatitis B virus (HBV) infection in a human, and encompasses includes the administration of an effective amount of L-FMAU, FTC and interferon.

### BACKGROUND OF THE INVENTION

Despite the existence of efficient vaccines, hepatitis B virus (HBV) infection remains a major public health problem worldwide with 400 million chronic carriers. These infected patients are exposed to a risk of developing liver cirrhosis and hepatocellular carcinoma (Lee, W. M. 1997, N. Engl. J. Med. 337:1733-1745). Currently, there are believed to be approximately 1.25 million chronic hepatitis B carriers just in the United States, with 200,000 people newly infected each year by contact with blood or body fluids.

Hepatitis B virus ("HBV") is second only to tobacco as a cause of human cancer. The mechanism by which HBV induces cancer is unknown, although it is postulated that it may directly trigger tumor development, or indirectly trigger tumor development through chronic inflammation, cirrhosis and cell regeneration associated with the infection.

The number of humans infected with HBV has reached epidemic levels. After a two to six month incubation period in which the host is unaware of the infection, HBV infection can lead to acute hepatitis and liver damage, which results in abdominal pain, jaundice, and elevated blood levels of certain enzymes. HBV can cause fulminant hepatitis, a rapidly progressive, often fatal form of the disease in which massive sections of the liver are destroyed. Patients typically recover from acute viral hepatitis. In some patients, however, high levels of viral antigen persist in the blood for an extended, or indefinite, period, causing a chronic infection. Chronic infections can lead to chronic persistent hepatitis. Patients infected with chronic persistent HBV are most common in developing countries. Chronic persistent hepatitis can cause fatigue, cirrhosis of the liver and hepatocellular carcinoma, a primary liver cancer. In western industrialized countries, high risk groups for HBV infection include those in contact with HBV carriers or their blood samples. The epidemiology of HBV is in fact very similar to that of acquired immunodeficiency syndrome, in fact, HBV infection is common among patients with AIDS or HIV-associated infections. However, HBV is more contagious than HIV.

To date, only three drugs have been approved by the FDA for the treatment of chronic HBV infection: interferon alpha, 3TC (Epivir, lamivudine) and adefovir dipivoxil (Hepsera^{®} Gilead Sciences).

### FDA Approved Drugs for HBV:

| Drug Name | Drug Class | Company | FDA Status |
|---|---|---|---|
| Intron A (interferon α-2b) | interferon | Schering-Plough | FDA-approved |
| 3TC (lamivudine; Epivir-HBV) | nucleoside analogue | GlaxoSmithKline | FDA-approved |
| Adefovir dipivoxil | nucleotide analogue | Gilead Sciences | FDA-approved |

### Interferon alpha

A manufactured form of interferon is used to treat hepatitis B. This treatment involves the administration of interferon by injection for about four months.

Not all patients respond to interferon, and sometimes retreatment is necessary. In clinical studies, only 45% of patients who were treated for hepatitis B with A (Interferon alpha-2b, recombinant, Schering Corporation) for Injection had no evidence of the hepatitis B virus in their blood over time. In addition, most patients have difficulty tolerating interferon treatment, which causes severe flu-like symptoms, weight loss, and lack of energy and stamina.

### 3TC

The (-)-enantiomer of BCH-189 (2',3'-dideoxy-3'-thiacytidine), also known as 3TC (Epivir, lamivudine) is an antiviral drug that is active against both HIV and HBV. It belongs to the class of drugs called nucleoside analog reverse transcriptase inhibitors (NRTT), which work by blocking production of the reverse transcriptase enzyme that HIV and HBV need in order to replicate. 3TC was originally developed for the treatment of HIV, however, researchers discovered that 3TC also works against the hepatitis B virus. In December 1998, the U.S. Food and Drug Administration (FDA) approved Epivir HBV for the treatment of hepatitis B virus infection.

Although 3TC efficiently inhibits HBV replication, the slow kinetics of viral elimination during 3TC therapy (Nowak, M., S. Bonhoeffer, et al. 1996. Proc. Natl. Acad. Sci. USA 93:4398-4402) and the spontaneous viral genome variability lead to the emergence of drug-resistant mutants which carry mutations affecting the reverse transcriptase (RT) domain (Mason, W. S., J. Cullen, et al. 1998. Virology 245:18-32. Nafa, S., S. Ahmed, et al. 2000. Hepatology 32:1078-1088; Melegari, M., P. P. Scaglioni, and J. R. Wands. 1998 Hepatology 27:628-633.; Seigneres, B., C. Pichoud, et al. 2000. J. Infect. Dis. 181:1221-1233). Approximately 50% of treated patients develop viral resistance after 3 years of treatment with 3TC (Leung, N. W., C. L. Lai, et al. 2001. Hepatology 33:1527-1532). Resistance to nucleoside analogs is associated with substitutions in the nucleic acid sequence of the polymerase gene causing changes in the amino acid sequence of the HBV RT, notably in the YMDD motif within the catalytic site. The most common polymerase variant is the rtL180M-plus-M204V change (according to the recent genotype-independent nomenclature for HBV drug-resistant variants) (Stuyver, L. J., S. A. Locarnini, et al. 2001. Hepatology 33:751-757) that associates a mutation in the catalytic site (rtM204V) with a compensatory mutation in the B domain of the RT (rtL180M) which provides a higher replication capacity to the catalytic site variant (Allen, M. I., M. Deslauriers, et al. 1998. Hepatology 27:1670-1677. Chayama, K., Y. Suzuki, et al. 1998. Hepatology 27:1711-1716. Melegari, M., P. P. Scaglioni, and J. R. Wands. 1998. Hepatology 27:628-633. Ono, S. K., N. Kato, et al. 2001. J. Clin. Investig. 107:449-455. Seigneres, B., S. Aguesse-Germon, et al. 2001. J. Hepatol. 34:114-122).

### Adefovir dipivoxil (Hepsera)

On September 20, 2002, the U.S. Food and Drug Administration approved adefovir dipivoxil for the treatment of chronic hepatitis B. HEPSERA™ is the tradename for adefovir dipivoxil, a diester prodrug of adefovir. Adefovir is an acyclic nucleotide analogue of adenosine monophosphate that inhibits the hepatitis B virus (HBV) DNA polymerase by competing with the natural substrate deoxyadenosine triphosphate and by causing DNA chain termination after its incorporation into viral DNA. The chemical name of adefovir dipivoxil is 9-[2-[bis[(pivaloyloxy)methoxy]phosphinyl]methoxyl-ethyl] adenine. Adefovir is phosphorylated to the active metabolite, adefovir diphosphate, by cellular kinases. See, for example, U.S. Patent Nos. 5,641,763 and 5,142,051, entitled, N-phosphonylmethoxyalkyl derivatives of pyrimidine and purine bases and a therapeutical composition therefrom with antiviral activity.

### (-)-FTC

β-L-FTC ((β-2-hydroxymethyl-5-(5-fluorocytosin-1-yl)-1,3-oxathiolane, Emtriva; emtricitabine) is approved for the treatment of HIV and currently in human clinical trials for the treatment of hepatitis B virus infection. The compound show strong activity against hepatitis B virus in duck models (Aguesse-Germon, S., S.-H, Liu, et al. Antimicrob. Agents Chemother. 1998, 42, 369-376; Seignéres, B., C. Pichoud, *et al.* **2000**) and woodchucks. In a woodchuck model of HBV, FTC was found to inhibit viral replication but not induce viral clearance. (Cullen, J. M., S. L. Smite, et al. Antimicrob. Agents Chemother. 1997, 41, 2076-2082; Korba, B. E., R. F. Schinazi, P., et al. Antimicrob. Agents Chemother. 2000, 44, 1757-60).

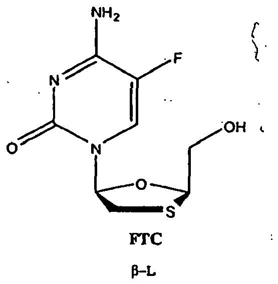

### L-FMAU

L-FMAU (clevudine) represent another promising candidate for the treatment of a hepatitis B virus infection based on *in vitro* models of HBV infection (enzymatic assays and tissue culture experiments), as well as in experimentally infected animal models of hepatitis B infection, such as in ducks (Aguesse-Germon, S., S.-H, Liu, et al. Antimicrob. Agents Chemother. 1998, 42, 369-376; Seignéres, B., C. Pichoud, *et al.,* 2000) and woodchucks. In the woodchuck model, L-FMAU was shown to decrease rapidly the number of infected hepatocytes (Zhu, Y., T. Yamamoto, et al. J. Virol. 2001, 75, 311-22). Yung Chi Cheng, Chung K. Chu and others first reported that 1-(2'-deoxy-2'-fluoro-β-L-arabinofuranosyl)-thymine (L-FMAU) exhibits superior activity against hepatitis B virus and Epstein Barr virus in 1994. See U.S. Patent Nos. 5,587,362; 5,567,688; 5,565,438 and 5,808,040 and International Patent Application published as WO 95/20595. International Patent Publication No. WO 01/72294 discloses the use of 2'-deoxy-2'-fluoro-β-L-arabinofuranosyl thymine (L-FMAU) for the treatment of hepatitis delta infections.

### Interferons

Interferon is a protein made naturally by the body to modulate the immune system and to regulate other cell functions. The main classes of interferons are interferon alpha, interferon beta, interferon gamma, interferon omega and interferon tau. Interferons can be modified to increase stability *in vivo,* such modifications include pegylation, or other means to enhance the stability of the molecule.

Examples of the interferon alpha class of interferons include interferon alpha-2a, interferon alpha-2b, pegylated interferon alpha-2a, pegylated interferon alpha-2b ROFERON®-A (interferon alpha-2a, Roche), PEGASYS® (pegylated interferon alpha-2a, Roche), INTRON®A (Interferon alpha-2b, Schering Corporation), PEG-INTRON® (pegylated Interferon alpha-2b, Schering Corporation), consensus interferon, INFERGEN (interferon alphacon-1) by InterMune, OMNIFERON (natural interferon) by Viragen, ALBUFERON by Human Genome Sciences, Oral Interferon Alpha by Amarillo Biosciences, and SuperFeron (natural human multi-subtype IFN-alpha, Genetrol, Inc.).

Other types of interferon include: interferon beta, interferon gamma, interferon tau, interferon omega, REBIF (interferon beta-la) by Ares-Serono, Omega Interferon by BioMedicine, interferon gamma-1b by InterMune, and HuFeron (human IFN-beta, Genetrol, Inc.).

### Combination Therapy

In order to better control viral replication and delay the emergence of virus-resistant mutants, it is critical to develop antiviral strategies based on combination therapy (Zoulim, F. 2001. Antivir. Chem. Chemother. 12(Suppl. 1):131-142), which may also prevent the selection of cross-resistant mutants, as was shown with human immunodeficiency virus (Chow, Y. K., M. S. Hirsch, et al. 1993. Nature 361:650-654; Snyder, S., D. Z. D'Argenio, et al. 2000. Antimicrob. Agents Chemother. 44:1051-1058.Villahermosa, M. L., et al..1997. Biochemistry 36:13223-13231). A few studies have focused on examination of the antiviral effect of the combination of polymerase inhibitors for the therapy of chronic hepadnavirus infection, using several experimental models (Korba, et al. 2000. Antivir. Res. 45:19-32; Colledge, D., S. Locamini, and T. Shaw. 1997. Hepatology 26:216-225; Colledge, et al. 2000. Antimicrob. Agents Chemother. 44:551-560). The development of combination therapy for hepatitis B infection is one current necessary strategies underway to further reduce the morbidity and mortality of chronic hepatitis B infection.

The effects of certain pyrimidine and purine nucleoside combinations in the duck hepatitis B virus infection model were evaluated by Seigneres et al., Antimicrobial Agents and Chemotherapy, 47(6):1842-1852 (2003) and Artiviral Research, 53(3), page A72. Combinations of amdoxovir (DAPD), emtricitabine ((-)-FTC) and clevudine (L-FMAU) have an enhanced antiviral effect.

Pichoud et al, Antiviral Research, 53 (3), page A72, discloses that IFN-γ monotherapy had only a transient effect on WHV replication. Delaney et al., Expert Opinion on Investigational Drugs, 11(2), pp 169-187, reviews some potential therapies for WHV.

Treatments for hepatitis B infection are also described in Lok and McMahon, AASLD Practice Guidelines, pp.1225-1241 (2001), including treatment with interferons. Eastern woodchucks chronically infected with the woodchuck hepatitis virus (WHV) were used as a model of HBV infection to study the antiviral effect of 1-(2-fluoro-5-methyl-β-L-arabinofuranosyl)-uracil (L-FMAU) and WHV surface antigen vaccine. The humoral and cellular immunity associated with the combination of L-FMAU and vaccine resembled that observed in self-limited WHV infection (Menne et al., J. Virology, 76(11):5305-5314 (2002)).

Jacquard et al. published a study of the multidrug treatment of chronic hepatitis B using L-FMAU and FTC, along with adenovirus delivery of recombinant interferon gamma. (Jacquard et al., "Evaluation and Combination of Clevudine and Emtricitabine with Adenovirus Mediated Delivery of Interferon Gamma in the Woodchuck Model of GBV Infection", Poster presented at the Boston AASLD meeting, Nov. 2-5, 2002. See also the Asilomar meeting "The Molecular Biology of Hepatitis B Virus" Sept 29 - Oct 3, 2002.) This study suggested that interferon did not enhance the effect of L-FMAU and FTC, however, the study was limited to woodchuck hepatitis virus in woodchucks, and not human hepatitis B in humans.

U.S. Patent No. 5,808,040 to the University of Georgia Research Foundation and Yale University discloses that L-FMAU can be administered in combination with FTC, 3TC, carbovir, acyclovir, interferon, AZT, DDI (2',3'-dideoxyinosine), DDC (2',3'-dideoxycytidine), L-DDC, L-F-DDC, and D4T.

International Patent Application No. PCT/US99/25673, published as WO 00/25797 discloses combinations and methods using such combinations for treating HBV infection and related conditions in humans. In particular, the compositions comprise a synergistically effective amount of β-2-hydroxymethyl-5-(5-fluorocytosin-1-yl)-1,3-oxathiolane (FTC) and one of penciclovir (2-amino-1,9-dihydro-9-[4-hydroxy-3-(hydroxymethyl)butyl]-6H-purin-6-one, also referred to as "PCV"), famciclovir, or 9-[2-(phosphonomethoxy)ethyl]adenine (PMEA, also referred to below as Bis-POM-PMEA or BP-PMEA). Alternatively, the compositions comprise a synergistically effective amount of 2'-fluoro-5-methyl-β-L-arabinofulanolyluridine (L-FMAU) and one of Penciclovir, 9-[2-(phosphono-methoxy)ethyl]adenine (PMEA) or a β-D-1,3-dioxolane nucleoside, such as DAPD. Alternatively, the compositions comprise a synergistically effective amount of a β-D-1,3-dioxolane nucleoside, such as DAPD and PMEA.

PCT Publication No. WO 98/23285 discloses a method for the treatment or prophylaxis of hepatitis B virus infections in a human or animal patient which comprises administering to the patient effective or prophylactic amounts of penciclovir (or a bioprecursor thereof such as famciclovir) and alpha-interferon.

U.S. Patent No. 5,990,093 and International Patent Publication Nos. WO 95/07086 and WO 96/40164, assigned to Emory University, disclose methods to treat HBV comprising administering β-L-2',3'-dideoxyadenosine in combination or alternation with 2-hydroxy-methyl-5-(5-fluorocytosin-1-yl)-1,3-oxathiolane; 2-hydroxymethyl-5-(cytosin-1-yl)-1,3-oxathiolane; 2'-fluoro-5-iodo-arabinosyluracil (FIAU); 2'-fluoro-5-ethyl-arabinosyluracil (FEAU), carbovir, or interferon.

U.S. Patent Nos. 5,703,058, 5,905,070, and 6,232,300 and International Patent Publication No. WO 96/22778 disclose (5-carboximido or 5-fluoro)-(2',3'-unsaturated or 3'-modified) pyrimidine nucleosides for the treatment of HTV or HBV. In particular, the patents and international patent publication disclose combination therapies for the treatment of HIV or HBV comprising a (5-carboximido or 5-fluoro)-(2',3'-unsaturated or 3'-modified) pyrimidine nucleoside in combination with one or more of 2-hydroxymethyl-5-(5-fluorocytosin-1-yl)-1,3-oxathiolane, 2-hydroxymethyl-5-(cytosin-1-yl)-1,3-oxathiolane; 9->4-(hydroxymethyl)-2-cyclopenten-1-yl-guanine (carbovir), 9-(2-hydroxy-ethoxy)methyl-guanine (acyclovir), interferon, 3'-deoxy-3'-azidothymidine (AZT), 2',3'-dideoxyinosine (DDI), 2',3'-dideoxycytidine (DDC), (-)-2'-fluoro-5-methyl-beta-L-ARAuridine (L-(-)-FMAU) and 2',3'-didehydro-2',3'-dideoxythymidine (D4T).

While advances have been made in the treatment of hepatitis B and in combination therapies, it is still not known which combinations are effective to optimally substantially reduce or eliminate the human hepatitis B viral load in the host. It has been observed that while certain hepatitis B agents and combinations can be augmented with additional therapies, in other cases, additional therapies provide no added benefit and can actually be detrimental to the patient's well being. The health care provider, therefore, needs information on beneficial and advantageous combinations of hepatitis B therapies so that he or she can proceed with optimal treatment of the patient.

In light of the fact that hepatitis B virus has reached epidemic levels worldwide, and has severe and often tragic effects on the infected patient, there remains a strong need to provide new effective pharmaceutical methods, uses and combinations to treat humans infected with the virus that have low toxicity to the host.

In addition, the treatment of chronic hepatitis B requires long-term administration of nucleoside analogs to clear infected hepatocytes, which encourages the development of drug resistant viral strains. Due to the selection of drug resistant mutants, new strategies of treatment for chronic hepatitis B are required.

Therefore, it is another object of the present invention to provide compositions and methods for the treatment of human patients or other hosts infected with HBV.

It is another object of the present invention to provide methods and compositions to treat infections of drug resistant strains of HBV.

### SUMMARY OF THE INVENTION

L-FMAU administered in combination and/or alternation with FTC and in combination and/or alternation with an interferon, such as interferon alpha or interferon gamma provides superior therapy in humans against hepatitis B virus. In one embodiment, the interferon is administered in the form of a protein, typically directly into the vein or artery. In an alternate embodiment of the invention, the interferon is administered in the form of a nucleic acid, gene or gene fragment thereof that is expressed by the host. The interferon nucleic acid can be delivered to the host "naked", i.e., without a vector, or alternatively, can be delivered via a vector, including but not limited to a viral vector including an adenovirus vector.

In a preferred embodiment, the β-L-FTC and the L-FMAU are at least 90% free of their opposite β-D-enantiomers. In a more preferred embodiment, β-L-FTC and LFMAU are independently at least 95%, 98% or 99% free of their opposite β-D-enantiomers.

In one embodiment, the interferon is interferon alpha, optionally, pegylated interferon alpha. In another embodiment, the interferon alpha is selected from the group, including, but not limited to: interferon alpha-2a, interferon alpha-2b, pegylated interferon alpha-2a, pegylated interferon alpha-2b ROFERON®-A (interferon alpha-2a, Roche), PEGASYS® (pegylated interferon alpha-2a, Roche), INTRON®A (Interferon alpha-2b, Schering Corporation), PEG-INTRON® (pegylated Interferon alpha-2b, Schering Corporation), consensus interferon, INFERGEN (interferon alphacon-1) by InterMune, OMNIFERON (natural interferon) by Viragen, ALBUFERON by Human Genome Sciences, Oral Interferon Alpha by Amarillo Biosciences, and SuperFeron (natural human multi-subtype IFN-alpha, Genetrol, Inc.). In an alternate embodiment, the interferon is interferon gamma. In yet another embodiment, the interferon is interferon beta, interferon omega or interferon tau. In another embodiment, the interferon is selected from the group, including, but not limited to: REBIF (interferon beta-1a) by Ares-Serono, Omega Interferon by BioMedicine, interferon gamma-1b by InterMune, and HuFeron (human IFN-beta, Genetrol, Inc.).

In one embodiment of the invention, the interferon is delivered in the form of a protein. In an alternate embodiment, the interferon is delivered in the form of a nucleic acid, gene or gene fragment that expresses the immunomodulator protein. In one particular embodiment of the present invention, the interferon is delivered in the form of a nucleic acid, gene or gene fragment thereof either "naked" or via a vector.

In general, during alternation therapy, an effective dosage of each agent is administered serially, whereas in combination therapy, effective dosages of two or more agents are administered together. The dosages will depend on such factors as absorption, bio-distribution, metabolism and excretion rates for each drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions. Examples of suitable dosage ranges can be found in the scientific literature and in the *Physicians Desk Reference.* Many examples of suitable dosage ranges for other compounds described herein are also found in public literature or can be identified using known procedures. These dosage ranges can be modified as desired to achieve a desired result.

### BRIEF DESCRIPTION OF THE FIGURES

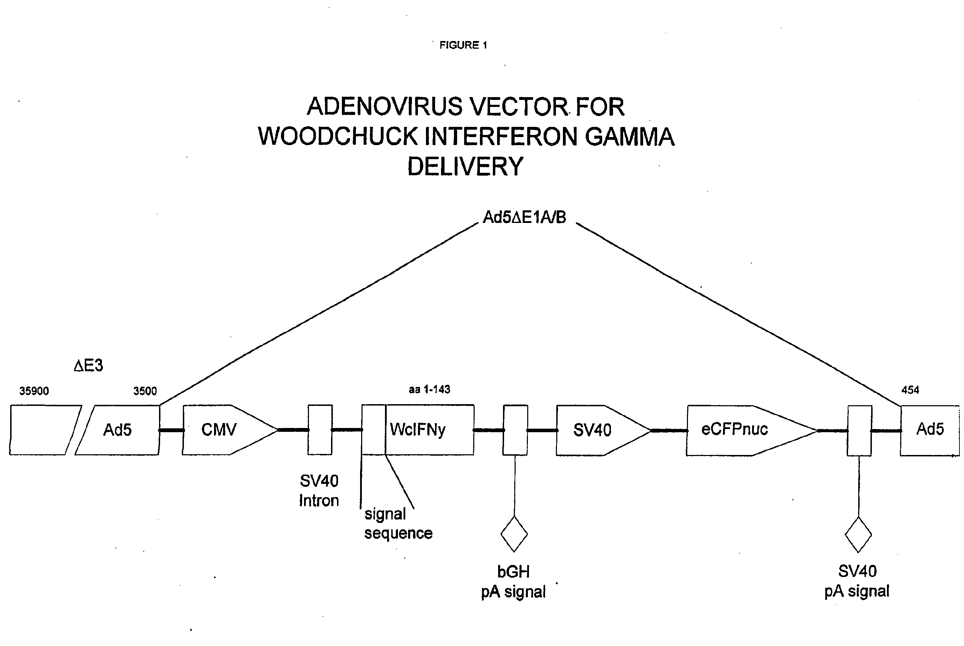

**Figure 1** is an illustration of a non-limiting example of an adenoviral vector comprising woodchuck interferon gamma suitable for delivery. Coding regions are depicted and defined.

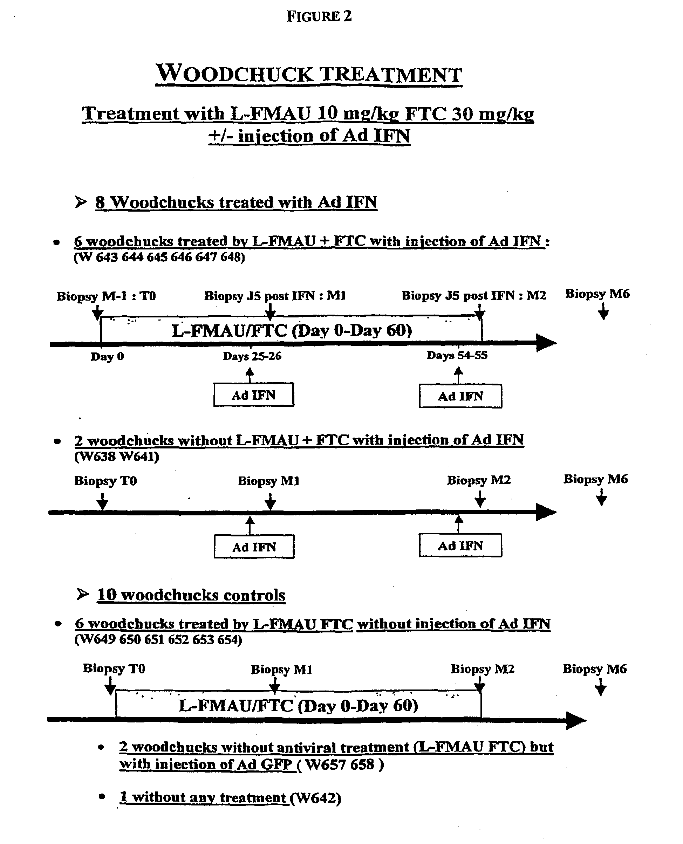

**Figure 2** is an illustration of a non-limiting schematic of treatment regimes of 1-(2-fluoro-5-methyl-β-L-arabinosyl) uracil (L-FMAU), and β-2-hydroxymethyl-5-(5-fluorocytosin-1-yl)-1,3-oxathiolane (FTC), with or without injections of an adenovirus vector for expressing woodchuck interferon gamma (Ad IFN) or an adenovirus vector expressing Green Fluorescent Protein (Ad GFP), given to representative woodchucks.

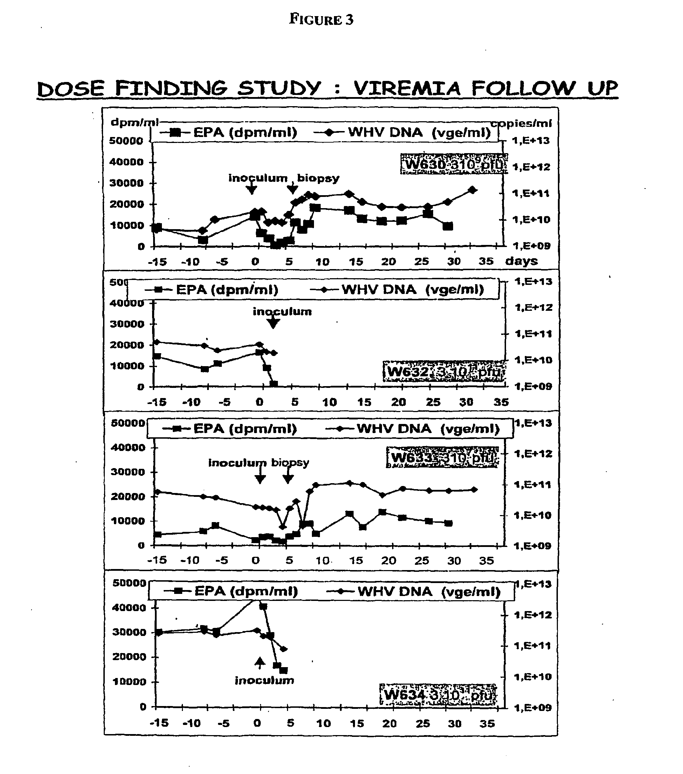

**Figure 3** shows four graphs depicting the amount of viral DNA (copies/ml) of woodchuck hepatitis B virus (WHV) over time (days) before and after injection of woodchucks with different doses (pfu) of adenovirus vector for expressing woodchuck interferon gamma (Ad IFN). Arrows indicate time of inoculation and time of biopsy. On the left Y axes, amount of viral plus strand DNA is shown, measured by endogenous polymerase assay (EPA, dpm/ml) as described in Example 3, plotted against time.

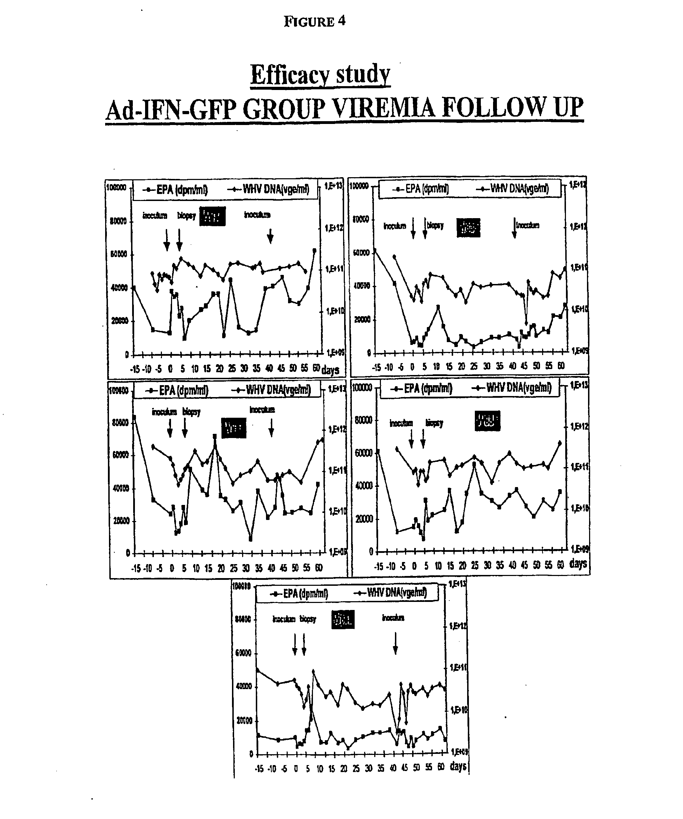

**Figure 4** shows five graphs depicting the amount of viral DNA (copies/ml) of woodchuck hepatitis B virus (WHV) over time (days) before and after injection of woodchucks with 3x10⁹ pfu of the complete adenovirus vector for expressing woodchuck interferon gamma (Ad IFN GFP). Arrows indicate time of inoculation and time of biopsy. On the left Y axes, amount of viral plus strand DNA is shown, measured by endogenous polymerase assay (EPA, dpm/ml) as described in Example 3, plotted against time. The data are described in more detail in Example 5.

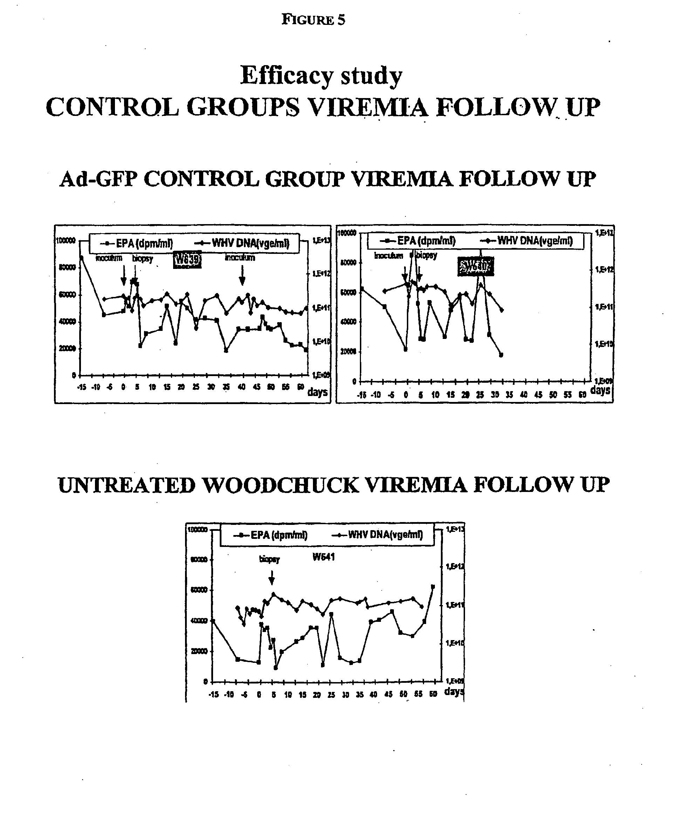

**Figure 5** is three graphs depicting the amount of viral DNA (copies/ml) of woodchuck hepatitis B virus (WHV) over time (days) before and after injection of woodchucks with 3x10⁹ pfu of adenovirus vector for expressing Green Fluorescent Protein (Ad GFP) (top two graphs) or an untreated control (bottom graph). Arrows indicate time of inoculation and time of biopsy. On the left Y axes, amount of viral plus strand DNA is shown, measured by endogenous polymerase assay (EPA, dpm/ml) as described in Example 3, plotted against time. The data are described in more detail in Example 5.

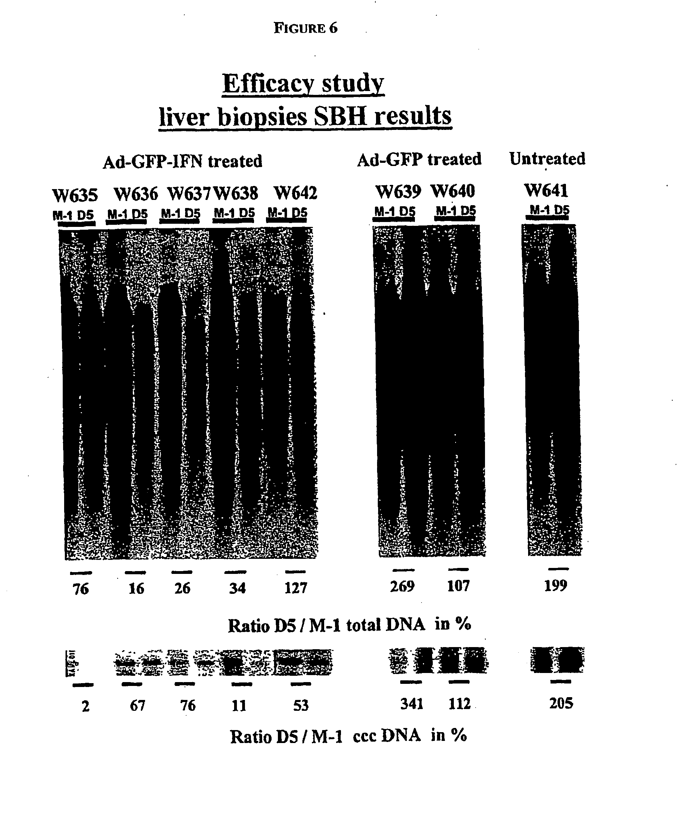

**Figure 6** is a copy of southern blots analyzing viral DNA found in liver biopsies of woodchucks infected with woodchuck hepatitis B virus (WHV), taken at 1 month before treatment (M-1) and day 5 post-injection (D5) from woodchucks injected with the complete adenovirus vector for expressing woodchuck interferon gamma (Ad IFN GFP), adenovirus vector for expressing Green Fluorescent Protein (Ad GFP), or not treated. Bottom blots are of covalently closed circular viral form of DNA (CCC) DNA. Numbers below the graphs demonstrate the ratio of DNA at DS/M-1 in percent.

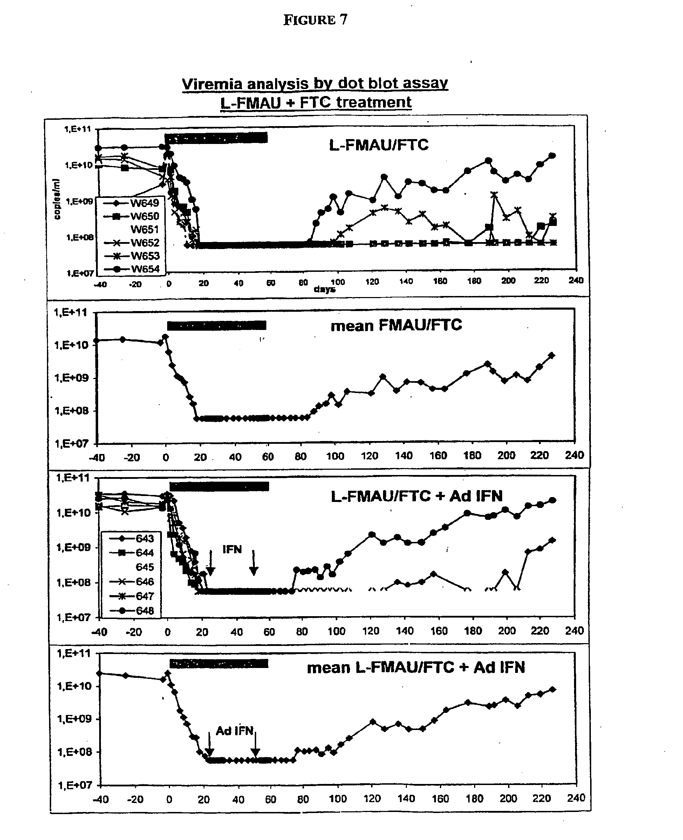

**Figure 7** shows graphs of viral concentration (copies/ml) of woodchuck hepatitis B virus over time (days) during and after treatment of animals with 1-(2-fluoro-5-methyl-β-L-arabinosyl) uracil (L-FMAU), and β-2-hydroxymethyl-5-(5-fluorocytosin-1-yl)-1,3-oxathiolane, Emtriva; emtricitabine (FTC), with or without injections of an adenovirus vector for expressing woodchuck interferon gamma (Ad IFN) as described in Example 6. The results were obtained using dot blot assays described in Example 3.

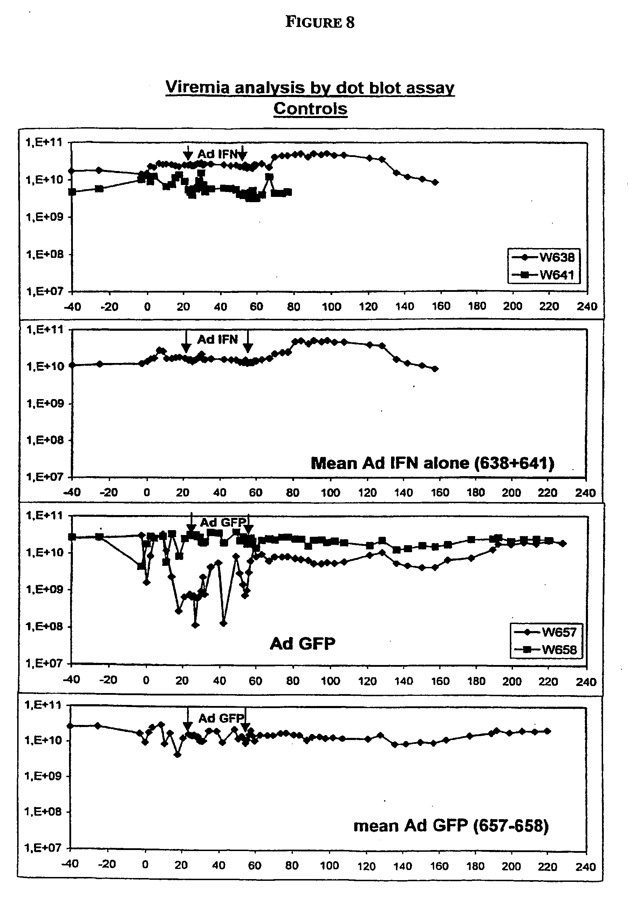

**Figure 8** shows graphs of viral concentration (copies/ml) of woodchuck hepatitis B virus over time (days) during and after treatment of animals with injections of adenovirus vector for expressing woodchuck interferon gamma (Ad IFN) or adenovirus vector for expressing Green Fluorescent Protein (Ad GFP) as described in Example 6. The results were obtained using dot blot assays described in Example 3. Control woodchucks treated with Ad IFN alone showed no significant variation in viremia.

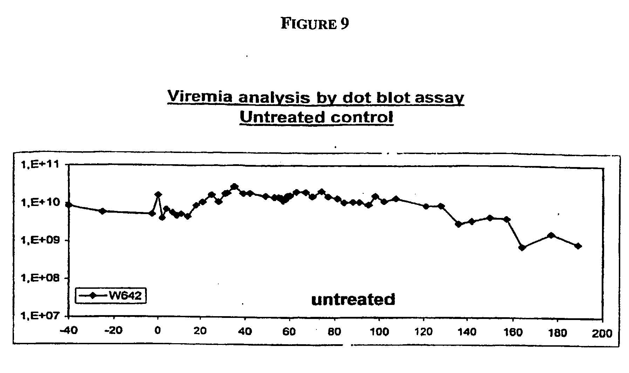

**Figure 9** is a graph of viral concentration (copies/ml) of woodchuck hepatitis B virus over time (days) in an untreated animal as described in Example 6. The results were obtained using dot blot assays described in Example 3.

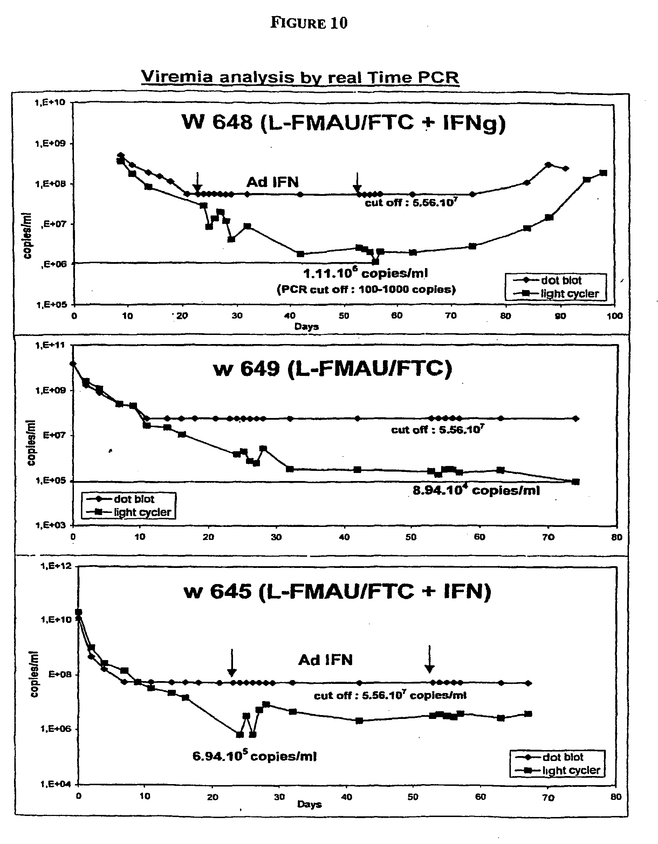

**Figure 10** is three graphs of viral load (copies/ml) of woodchuck hepatitis B virus over time (days) during and after treatment of animals with 1-(2-fluoro-5-methyl-β-L-arabinosyl) uracil (L-FMAU), and β-2-hydroxymethyl-5-(5-fluorocytosin-1-yl)-1,3-oxathiolane, Emtriva; emtricitabine (FTC), with or without injections of an adenovirus vector for expressing woodchuck interferon gamma (IFN), measured using real-time PCR as described in Example 3.

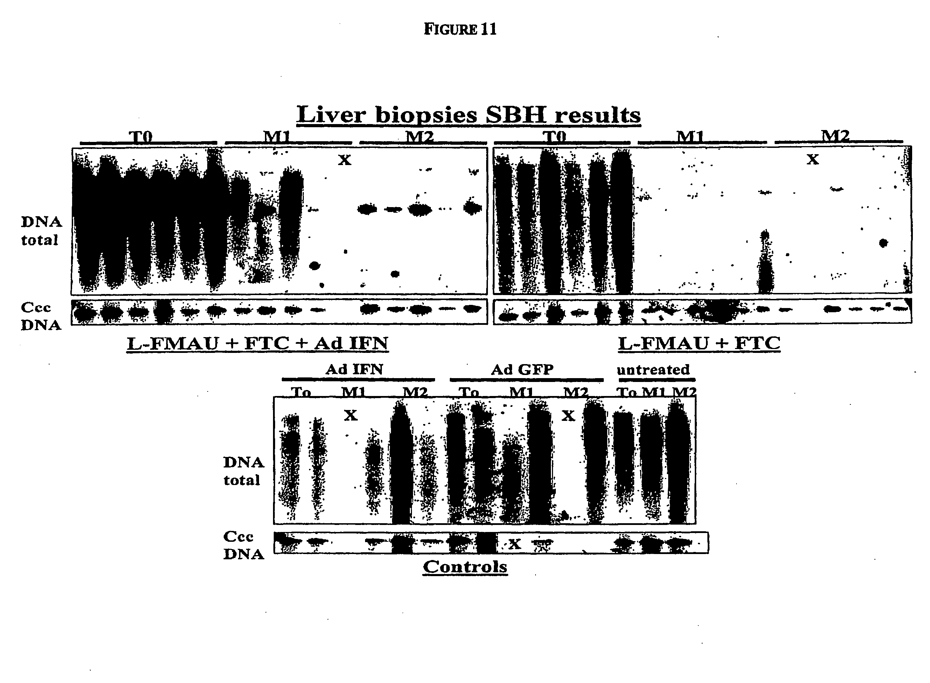

**Figure 11** is a copy of southern blots analyzing viral DNA found in liver biopsies of woodchucks infected with woodchuck hepatitis B virus (WHV), taken just prior to therapy (TO), at 1 month into treatment (M1) and at two months into treatment (M2) from woodchucks treated with 1-(2-fluoro-5-methyl-5-L-arabinosyl) uracil (L-FMAU), β-2-hydroxymethyl-5-(5-fluorocytosin-1-yl)-1,3-oxathiolane, Emtriva; emtricitabine (FTC), adenovirus vector for expressing woodchuck interferon gamma (Ad IFN), adenovirus vector for expressing Green Fluorescent Protein (Ad GFP), or no treatment. Bottom blots are of covalently closed circular viral form of DNA (CCC) DNA. Numbers below the graphs demonstrate the ratio of DNA at D5/M-1 in percent..

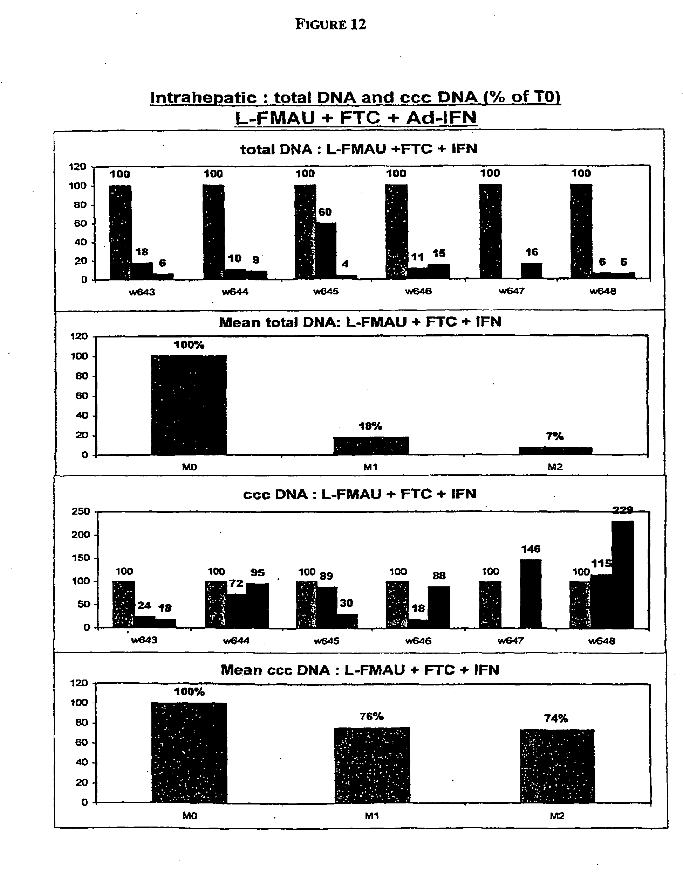

**Figure 12** are graphs depicting the percent of woodchuck hepatitis B virus (WHV) viral DNA and covalently closed circular viral form of DNA (CCC) DNA in animals treated with 1-(2-fluoro-5-methyl-β-L-arabinosyl) uracil (L-FMAU), β-2-hydroxymethyl-5-(5-fluorocytosin-1-yl)-1,3-oxathiolane, Emtriva; emtricitabine (FTC), or an adenovirus vector for expressing woodchuck interferon gamma (IFN), indicating the depletion of virus. M0 indicates samples taken just prior to therapy, M1 indicates samples taken at 1 month into treatment, and M2 indicates samples taken at two months into treatment.

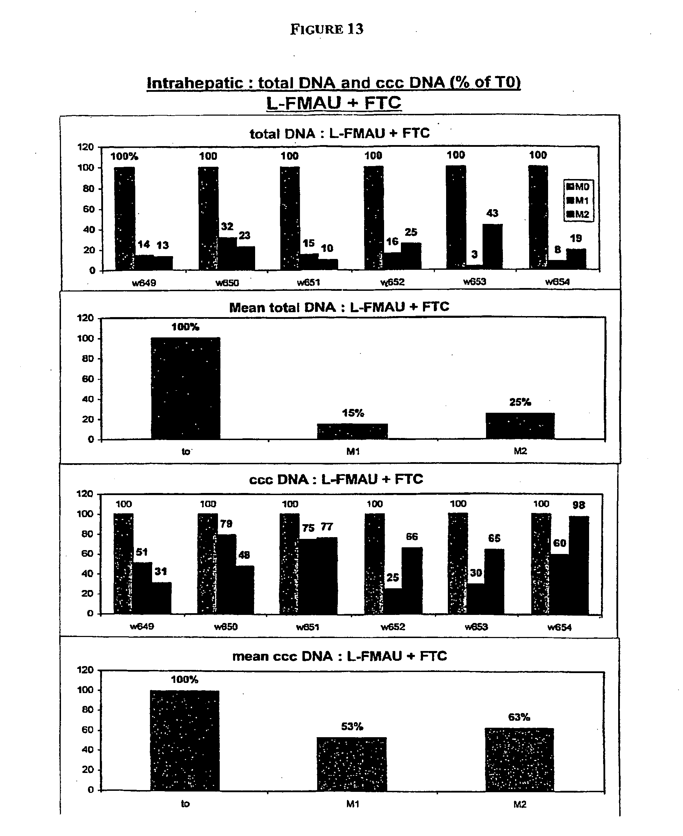

**Figure 13** are graphs depicting the percent of woodchuck hepatitis B virus (WHV) viral DNA and covalently closed circular viral form of DNA (CCC) DNA in animals treated with 1-(2-fluoro-5-methyl-β-L-arabinosyl) uracil (L-FMAU), and β-2-hydroxymethyl-5-(5-fluorocytosin-1-yl)-1,3-oxathiolane, Emtriva; emtricitabine (FTC), indicating the depletion of virus. T0 indicates samples taken just prior to therapy, M1 indicates samples taken at 1 month into treatment, and M2 indicates samples taken at two months into treatment..

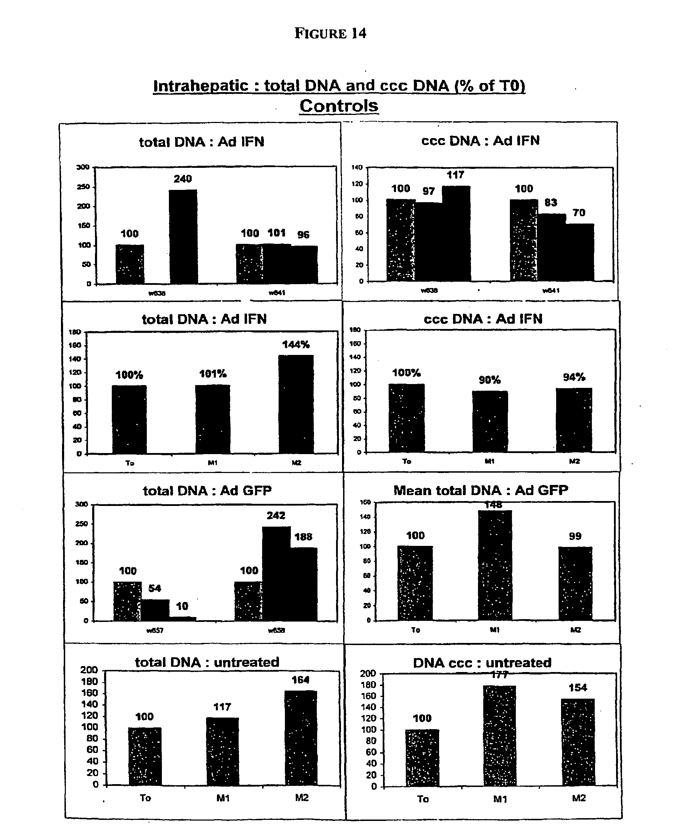

**Figure 14** are graphs depicting the percent of woodchuck hepatitis B virus (WHY) viral DNA and covalently closed circular viral form of DNA (CCC) DNA in animals treated with injections of adenovirus vector for expressing woodchuck interferon gamma (Ad IFN), adenovirus vector for expressing Green Fluorescent Protein (Ad GFP), or untreated. T0 indicates samples taken just prior to therapy, M1 indicates samples taken at 1 month into treatment, and M2 indicates samples taken at two months into treatment.

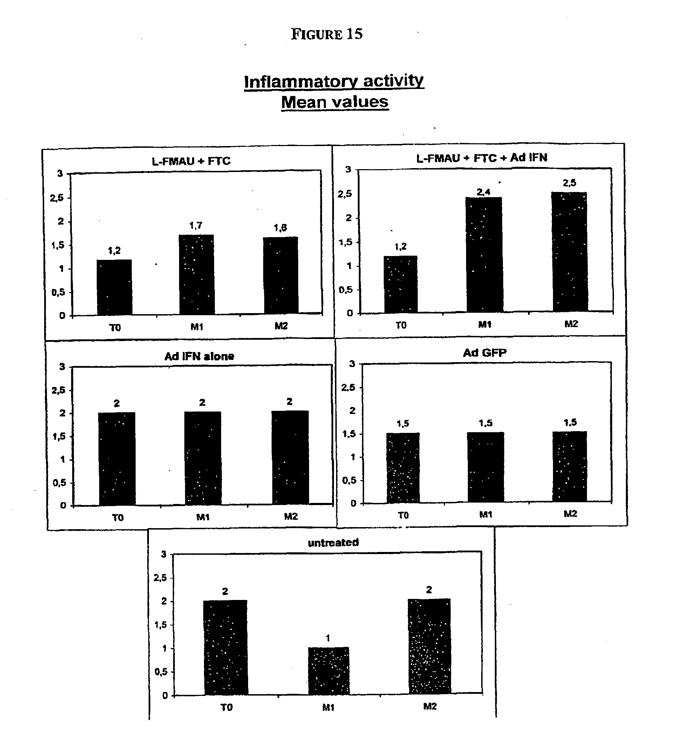

**Figure 15** are graphs depicting the amount of inflammatory activity (Metavir score) as described in Example 2, after treatment with with 1-(2-fluoro-5-methyl-β-L-arabinosyl) uracil (L-FMAU), β-2-hydroxymethyl-5-(5-fluorocytosin-1-yl)-1,3. oxathiolane, Emtriva; emtricitabine (FTC), injections of adenovirus vector for expressing woodchuck interferon gamma (Ad IFN), adenovirus vector for expressing Green Fluorescent Protein (Ad GFP), or untreated controls.

### DETAILED DESCRIPTION OF THE INVENTION

The invention is as set out in the appended claims.

L-FMAU administered in combination and/or alternation with FTC and in combination and/or alternation with an interferon, such as interferon alpha or interferon gamma provides superior therapy in humans against hepatitis B virus. In one embodiment, the interferon is administered in the form of a protein, typically directly into the vein or artery. In an alternate embodiment of the invention, the interferon is administered in the form of a nucleic acid, gene or gene fragment thereof that is expressed by the host. The interferon nucleic acid can be delivered "naked" or via a vector, using techniques known to those of ordinary skill in the art.

In a preferred embodiment, the β-L-FTC and the L-FMAU are at least 90% free of their opposite β-D-enantiomers. In a more preferred embodiment, β-L-FTC and LFMAU are independently at least 95%, 98% or 99% free of their opposite β-D-enantiomers.

In a preferred embodiment, the immunomodulator is an interferon. In one embodiment, the interferon is interferon alpha, optionally, pegylated interferon alpha. In another embodiment, the interferon alpha is selected from the group, including, but not limited to: interferon alpha-2a, interferon alpha-2b, pegylated interferon alpha-2a, pegylated interferon alpha-2b ROFERON®-A (interferon alpha-2a, Roche), PEGASUS® (pegylated interferon alpha-2a, Roche), INTRON®A (Interferon alpha-2b, Schering Corporation), PEG-INTRON® (pegylated Interferon alpha-2b, Schering Corporation), consensus interferon, INFERGEN (interferon alphacon-1) by InterMune, OMNIFERON (natural interferon) by Viragen, ALBUFERON by Human Genome Sciences, Oral Interferon Alpha by Amarillo Biosciences, and SuperFeron (natural human multi-subtype IFN-alpha, Genetrol, Inc.). In an alternate embodiment, the interferon is interferon gamma. In yet another embodiment, the interferon is interferon beta, interferon omega or interferon tau. In another embodiment, the interferon alpha is selected from the group, including, but not limited to: REBIF (interferon beta-la) by Ares-Serono, Omega Interferon by BioMedicine, interferon gamma-1b by InterMune, and HuFeron (human IFN-beta, Genetrol, Inc.).

In one embodiment of the invention, the interferon is delivered in the form of a protein. In an alternate embodiment, the interferon is delivered in the form of a nucleic acid, gene or gene fragment that expresses the protein. In one particular embodiment of the present invention, the interferon is delivered in the form of a nucleic acid, gene or gene fragment thereof, and the delivery is aqccomplished with a viral vector or via "naked" delivery.

In general, during alternation therapy, an effective dosage of each agent is administered serially, whereas in combination therapy, effective dosages of two or more agents are administered together. The dosages will depend on such factors as absorption, bio-distribution, metabolism and excretion rates for each drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions. Examples of suitable dosage ranges can be found in the scientific literature and in the *Physicians Desk Reference.* Many examples of suitable dosage ranges for other compounds described herein are also found in public literature or can be identified using known procedures. These dosage ranges can be modified as desired to achieve a desired result.

### I. Definitions

The term "alkyl", as used herein, unless otherwise specified, refers to a saturated straight, branched, or cyclic, primary, secondary or tertiary hydrocarbon of typically C₁ to C₁₀, and specifically includes methyl, trifluoromethyl, CCl₃, CFCl₂, CF₂Cl, ethyl, CH₂CF₃, CF₂CF₃, propyl, isopropyl, cyclopropyl, butyl, isobutyl, *t*-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl. The term includes both substituted and unsubstituted alkyl groups. Moieties with which the alkyl group can be substituted are selected from the group consisting of halogen (fluoro, chloro, bromo or iodo), hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

The term "lower alkyl", as used herein, and unless otherwise specified, refers to a C₁ to C₄ saturated straight, branched, or if appropriate, a cyclic (for example, cyclopropyl) alkyl group, including both substituted and unsubstituted forms. Unless otherwise specifically stated in this application, when alkyl is a suitable moiety, lower alkyl is preferred. Similarly, when alkyl or lower alkyl is a suitable moiety, unsubstituted alkyl or lower alkyl is preferred.

The term "aryl", as used herein, and unless otherwise specified, refers to phenyl, biphenyl, or naphthyl, and preferably phenyl. The term includes both substituted and unsubstituted moieties. The aryl group can be substituted with one or more moieties selected from the group consisting of halogen (fluoro, chloro, bromo or iodo), hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991.

The term "acyl" refers to a carboxylic acid ester in which the non-carbonyl moiety of the ester group is selected from straight, branched, or cyclic alkyl or lower alkyl, alkoxyalkyl including methoxymethyl, aralkyl including benzyl, aryloxyalkyl such as phenoxymethyl, aryl including phenyl optionally substituted with halogen (e.g., F, Cl, Br or I), C₁ to C₄ alkyl or C₁ to C₄ alkoxy, sulfonate esters such as alkyl or aralkyl sulphonyl including methanesulfonyl, the mono, di or triphosphate ester, trityl or , monomethoxytrityl, substituted benzyl, trialkylsilyl (e.g. dimethyl-t-butylsilyl) or diphenylmethylsilyl. Aryl groups in the esters optimally comprise a phenyl group. The term "lower acyl" refers to an acyl group in which the non-carbonyl moiety is lower alkyl.

The term "substantially pure form" is used throughout the specification to describe a compound which includes approximately 90% or greater, alternatively at least 95%, 96%, 97%, 98%, or 99% or more of a single enantiomer of that compound. When a nucleoside of a particular configuration (D or L) is referred to in this specification, it is presumed that the nucleoside is administered in substantially pure form.

The term "immunomodulator," as used herein, refers to a chemokine or cytokine that regulates either directly or indirectly an immune response. Non-limiting examples of immunomodulators include TH1 cytokines, and in particular, interferon, interferon-α, purified interferon-a, interferon-a2a, interferon-a2b, interferon-β, interferon-γ, consensus interferon, pegylated interferon, pegylated interferon-a, granulocyte macrophage colony-stimulating factor, interleukin, interleukin-2, and interleukin-12. In a preferred embodiment, the immunomodulator is interferon, and even more preferably interferons-γ.

The term "host," as used herein, refers to a unicellular or multicellular organism in which the virus can replicate, including cell lines and animals, and preferably a human. As set out in the claims, the invention is concerned with the treatment or prophylaxis of HBV in humans. Alternatively, the host can be carrying a part of the viral genome, whose replication or function can be altered by the compounds of the present invention. The term host specifically refers to infected cells, cells transfected with all or part of the viral genome and animals, in particular, primates (including chimpanzees) and humans. In most animal applications of the present invention, the host is a human patient. Veterinary applications, in certain indications, however, are clearly anticipated by the present invention.

### II. Pharmaceutically Acceptable Salts and Prodrugs

In cases where any of the compounds as disclosed herein are sufficiently basic or acidic to form stable nontoxic acid or base salts, administration of the compound as a pharmaceutically acceptable salt may be appropriate. Examples of pharmaceutically acceptable salts are organic acid addition salts formed with acids, which form a physiological acceptable anion, for example, tosylate, methanesulfonate, acetate, citrate, malonate, tartarate, succinate, benzoate, ascorbate, α-ketoglutarate and α-glycerophosphate. Suitable inorganic salts may also be formed, including, sulfate, nitrate, bicarbonate and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

"Pharmaceutically acceptable prodrugs" refer to a compound that is metabolized, for example hydrolyzed or oxidized, in the host to form the compound of the present invention. Typical examples of prodrugs include compounds that have biologically labile protecting groups on a functional moiety of the active compound. The invention relates to specific prodrugs as set out in the claims. Pharmaceutically acceptable salts include those derived from pharmaceutically acceptable inorganic or organic bases and acids. Suitable salts include those derived from alkali metals such as potassium and sodium, alkaline earth metals such as calcium and magnesium, among numerous other acids well known in the pharmaceutical art. The compounds of this invention either possess antiviral activity, or are metabolized to a compound that exhibits such activity.

Any of the nucleosides described herein can be administered as a nucleotide prodrug to increase the activity, bioavailability, stability or otherwise alter the properties of the nucleoside. A number of nucleotide prodrug ligands are known. In general, alkylation, acylation or other lipophilic modification of the hydroxyl group of the compound or of the mono, di or triphosphate of the nucleoside will increase the stability of the nucleotide. Examples of substituent groups that can replace one or more hydrogens on the phosphate moiety are alkyl, aryl, steroids, carbohydrates, including sugars, 1,2-diacylglycerol and alcohols. Many are described in R. Jones and N. Bischofberger, Antiviral Research, 27 (1995) 1-17. Any of these can be used in combination with the disclosed nucleosides to achieve a desired effect.

Any of the compounds which are described herein for use in combination and/or alternation therapy can be administered as an acylated prodrug, wherein the term acyl refers to a carboxylic acid ester in which the non-carbonyl moiety of the ester group is selected from straight, branched, or cyclic alkyl or lower alkyl, alkoxyalkyl including methoxymethyl, aralkyl including benzyl, aryloxyalkyl such as phenoxymethyl, aryl including phenyl optionally substituted with halogen, C₁ to C₄ alkyl or C₁ to C₄ alkoxy, sulfonate esters such as alkyl or aralkyl sulphonyl including methanesulfonyl, the mono, di or triphosphate ester, trityl or monomethoxytrityl, substituted benzyl, trialkylsilyl (e.g. dimethyl-t-butylsilyl).

The active nucleoside or other hydroxyl containing compound can also be provided as an ether lipid (and particularly a 5'-ether lipid or a 5'-phosphoether lipid for a nucleoside), as disclosed in the following references, which are incorporated by reference herein: Kucera, L.S., N. Iyer, et al. AIDS Res. Hum. Retro Viruses. 6:491-501; Piantadosi, C., J. Marasco C.J., et al. J. Med. Chem. 34:1408.1414; Hosteller, K.Y., D.D. Richman, et al. Antimicrob. Agents Chemother. 36:2025.2029; Hostetler, K.Y., L.M. Stuhmiller, et al. J. Biol. Chem. 265:61127.

Nonlimiting examples of U.S. patents that disclose suitable lipophilic substituents that can be covalently incorporated into the nucleoside or other hydroxyl or amine containing compound, preferably at the 5'-OH position of the nucleoside or lipophilic preparations, include U.S. Patent Nos. 5,149,794 (Sep. 22, 1992, Yatvin et al.); 5,194,654 (Mar. 16, 1993, Hostetler et al., 5,223,263 (June 29, 1993, Hostetler et al.); 5,256,641 (Oct. 26, 1993, Yatvin et al.); 5,411,947 (May 2, 1995, Hostetler et al.); 5,463,092 (Oct. 31, 1995, Hostetler et al.); 5,543,389 (Aug. 6, 1996, Yatvin et al.); 5,543,390 (Aug. 6, 1996, Yatvin et al.); 5,543,391 (Aug. 6, 1996, Yatvin et al.); and 5,554,728 (Sep. 10, 1996; Basava et al.), all of which are incorporated herein by reference. Foreign patent applications that disclose lipophilic substituents that can be attached to the nucleosides of the present invention, or lipophilic preparations, include WO 89/02733, W0 90/00555, W0 91/16920, W0 91/18914, W0 93/00910, W0 94/26273, W0 96/15132, EP 0 350 287, EP 93917054.4, and W0 91/19721.

Nonlimiting examples of nucleotide prodrugs are described in the following references: Ho, D.H.W. (1973) Cancer Res. 33, 2816-2820; Holy, A. (1993) Isopolar phosphorous-modified nucleotide analogues," In: De Clercq (Ed.), Advances in Antiviral Drug Design, Vol. I, JAI Press, pp. 179-231; Hong, C.I., Nechaev, A., and West, C.R. (1979a) Biochem. Biophys. Rs. Commun. 88, 1223-1229; Hong, C.I., Nechaev, A., et al. (1980) J. Med. Chem. 28, 171-177; Hosteller, K.Y., Stuhmiller, L.M. et al. J. Biol. Chem. 265, 6112-6117; Hosteller, K.Y., Carson, D.A. and Richman, D.D. (1991) J. Biol Chem. 266, 11714-11717; Hosteller, K.Y., Korba, B. et al. (1994a) Antiviral Res. 24, 59-67; Hosteller, K.Y., Richman, D.D., et al. (1994b) Antimicrobial Agents Chemother. 38, 2792-2797; Hunston, R.N., Jones, A.A. et al. (1984) J. Med. Chem. 27, 440-444; Ji, Y.H., et al. (1990); J. Med. Chem. 33 2264-2270; Jones, A.S., McGuigan, C., et al. (1984) J. Chem. Soc. Perkin Trans. I, 1471-1474; Juodka, B.A. and Smrt, J. (1974) Coll. Czech. Chem. Comun. 39, 363-968; Kataoka, S., Imai, J., et al. (1989) Nucleic Acids Res. Sym. Ser. 21, 1-2; Kataoka, S., Uchida, Heterocycles 32, 1351-1356; Kinchington, D., Harvey, J.J., et al. (1992) Antiviral Chem. Chemother. 3, 107-112; Kodama, K., Morozumi, M., et al. (1989) Jpn. J. Cancer Res. 80, 679-685; Korty, M. and Engels, J. (1979) Naunyn-Schmiedeberg's Arch. Pharmacol. 310, 103-111; Kumar, A., Goe, P.L., et al. (1990) J. Med. Chem, 33, 2368-2375; LeBec, C., and Huynh-Dinh, T. (1991) Tetrahedron Lett. 32, 6553-6556; Lichtenstein, J., Barner, H.D. and Cohen, S.S. (1960) J. Biol. Chem. 235, 457-465; Lucthy, J., Von Daeniken, A., et al. (1981) Mitt. Geg. Lebensmittelunters. Hyg. 72, 131-133 (Chem. Abstr. 95, 127093); McGigan, C. Tollerfield, S.M. and Riley, P.a. (1989) Nucleic Acids Res. 17, 6065-6075; McGuigan, C., Devine, K.G., et al. (1990a) Antiviral Chem. Chemother. 1107-113; McGuigan, C., O'Connor, et al. (1990b) Antiviral Chem. Chemother. 1, 355-360; McGuigan, C., Nicholls, S.R., et al. (1990c) Antiviral Chem. Chemother. 1, 25-33; McGuigan, C., Devin, K.G., et al. (1991) "Antiviral Res. 15, 255-263; McGuigan, C., Pathirana, RN., Balzarini, J. and DeClercq, E. (1993b) J. Med. Chem. 36, 1048-1052. Antiviral Chem. Chemother. 5, 271-277; Meyer, R. B., Jr., Shuman, D.A. and Robins, R.K. (1973) Tetrahedron Lett. 269-272; Nagyvary, J. Gohil, R.N., Kirchner, C.R. and Stevens, J.D. (1973) BioChem. Biophys. Res. Commun. 55, 1072-1077; Namane, A. Gouyette, C., et al. (1992) J. Med Chem. 35, 3039-3044; Nargeot, J. Nerbonne, et al. (1983) Natl. Acad Sci. U.S.A. 80, 2395-2399; Nelson, K.A., Bentrude, W.G. et al. (1987) J. Am. Chem. Soc. 109, 4058-4064; Nerbonne, J.M., Richard, S., Nargeot, J. and Lester, H.A. (1984) Nature 301, 74-76; Neumann, J.M., Herv, M., et al. (1989) J. Am. Chem. Soc. 111, 4270-4277; Ohno, R., Tatsumi, N., et al. (1991) Oncology 48, 451-455. Palomino, E., Kessle, D. and Horwitz, J.P. (1989) J. Med. Chem. 32, 22-625; Perkins, R.M., Barney, S. et al. (1993) Antiviral Res. 20 (Suppl. I). 84; Piantadosi, C., Marasco, C.J., Jr., et al. (1991) J. Med. Chem. 34, 1408-1414; Pompon, A., Lefebvre, I., Imbach, J.L., Kahn, S. and Farquhar, D. (1994). Antiviral Chem Chemother. 5, 91-98; Postemark, T. (1974) Annu. Rev. Pharmacol. 14, 23-33; Prisbe, E.J., Martin, J.C.M., et al. (1986) J. Med. Chem. 29, 671-675; Pucch, F., Gosselin, G., et al. (1993) Antiviral Res. 22, 155-174; Pugaeva, V.P., Klochkeva, S.I., Mashbits, F.D. and Eizengart, R.S. (1969) Gig. Trf. Prof. Zabol. 14, 47-48 (Chem. Abstr. 72, 212); Robins, R.K. (1984) Pharm. Res. 11-18; Rosowsky, A., Kim. S.H., Ross and J. Wick, M.M. (1982) J. Med. Chem. 25, 171-178; Ross, W. (1961) BioChem. Pharm. 8, 235-240; Ryu, E.K., Ross, R.J. Matsushita, T., et al. (1982). J. Med. Chem. 25, 1322-1329; Saffhill, R. and Hume, W.J. (1986) Chem. Biol. Interact. 57, 347-355; Saneyoshi, M., Morozumi, M., et al. (1980) Chem Pharm. Bull. 28, 2915-2923; Sastry, J.K., Nehete, P.N., et al. (1992) Mol. Pharmacol. 41, 441-445; Shaw, J.P., Jones, R.J. et al. (1994) "Oral bioavailability of PMEA from PMEA prodrugs in male Sprague-Dawley rats." 9th Annual AAPS Meeting. San Diego, CA (Abstract). Shuto, S., Ueda, S., Imamura, S., Fukukawa, K. Matsuda, A. and Ueda, T. (1987) Tetrahedron Lett. 28, 199-202; Shuto, S. Itoh, H., et al.. (1988) Pharm. Bull. 36, 209-217. An example of a useful phosphate prodrug group is the S-acyl-2-thioethyl group, also referred to as "SATE".

### III. Pharmaceutical Compositions

humans suffering from effects caused by HBV or who have been exposed to hepatitis B virus, can be treated by administering to the patient an effective amount of L-FMAU and β-L-FTC, in combination and/or alternation with an interferon as set out in the claims. The active materials can be administered by any appropriate route, for example, orally, parenterally, enterally, intravenously, intradermally, subcutaneously, topically, nasally, rectally, in liquid, or solid form.

The active compounds are included in the pharmaceutically acceptable carrier or diluent in an amount sufficient to deliver to a patient a therapeutically effective amount of compound to inhibit viral replication *in vivo,* especially HBV replication, without causing serious toxic effects in the treated patient. By "inhibitory amount" is meant an amount of active ingredient sufficient to exert an inhibitory effect as measured by, for example, an assay such as the ones described herein.

A preferred dose of the compound for all the above-mentioned conditions will be in the range from about 1 to 50 mg/kg, preferably 1 to 20 mg/kg, of body weight per day, more generally 0.1 to about 100 mg per kilogram body weight of the recipient per day. The effective dosage range of the pharmaceutically acceptable derivatives can be calculated based on the weight of the parent nucleoside to be delivered. If the derivative exhibits activity in itself, the effective dosage can be estimated as above using the weight of the derivative, or by other means known to those skilled in the art.

The compounds are conveniently administered in unit any suitable dosage form, including but not limited to one containing 7 to 3000 mg, preferably 70 to 1400 mg of active ingredient per unit dosage form. An oral dosage of 50 to 1000 mg is usually convenient.

Ideally, at least one of the active ingredients, though preferably the combination of active ingredients, should be administered to achieve peak plasma concentrations of the active compound of from about 0.2 to 70 mM, preferably about 1.0 to 10 mM. This may be achieved, for example, by the intravenous injection of a 0.1 to 10 % solution of the active ingredient, optionally in saline, or administered as a bolus of the active ingredient.

The concentration of active compound in the drug composition will depend on absorption, distribution, metabolism and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

A preferred mode of administration of FTC and L-FMAU is oral. Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible bind agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

The compounds can be administered as a component of an elixir, suspension, syrup, wafer, chewing gum or the like. A syrup may contain, in addition to the active compounds, sucrose as a sweetening agent and certain preservatives, dyes and colorings and flavors.

The compounds or their pharmaceutically acceptable derivative or salts thereof can also be mixed with other active materials that do not impair the desired action, or with materials that supplement the desired action, such as antibiotics, anti-fungals, antiinflammatories, protease inhibitors, or other nucleoside or non-nucleoside antiviral agents, as discussed in more detail above. Solutions or suspensions used for parental, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

If administered intravenously, preferred carriers are physiological saline or phosphate buffered saline (PBS).

If administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

If rectally administered in the form of suppositories, these compositions may be prepared by mixing the drug with a suitable non-initiating excipient, such as cocoa butter, synthetic glyceride esters of polyethylene glycols, which are solid at ordinary temperatures, but liquefy and/or dissolve in the rectal cavity to release the drug.

In one embodiment, one or more of the active compounds are prepared with carriers that will protect the compound against rapid elimination from the body, such as a controlled release formulation, including implants and micro-encapsulated delivery systems. Biodegradable, biocompatible polymers can be used, such as ethylene vinyl acetate, polyanhydrides, polyglycolic acid, collagen, polyorthoesters and polylactic acid. Methods for preparation of such formulations will be apparent to those skilled in the art. The materials can also be obtained commercially from Alza Corporation.

Liposomal suspensions (including liposomes targeted to infected cells with monoclonal antibodies to viral antigens) are also preferred as pharmaceutically acceptable carriers. These may be prepared according to methods known to those skilled in the art, for example, as described in U.S. Patent No. 4,522,811 For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its monophosphate, diphosphate, and/or triphosphate derivatives is then introduced into the container. The container is then swirled by hand to free lipid material from the sides of the container and to disperse lipid aggregates, thereby forming the liposomal suspension.

Compositions according to the present invention can be administered in combination and/or alternation with one or more other antiviral, anti-HIV, anti-HBV, anti-HCV or anti-herpetic agent or interferon, anti-cancer, antiproliferative or antibacterial agents, including other compounds of the present invention. Certain compounds according to the present invention may be effective for enhancing the biological activity of certain agents according to the present invention by reducing the metabolism, catabolism or inactivation of other compounds and as such, are co-administered for this intended effect.

In general, during alternation therapy, an effective dosage of each agent is administered serially, whereas in combination therapy, effective dosages of two or more agents are administered together. The dosages will depend on such factors as absorption, bio-distribution, metabolism and excretion rates for each drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions. Examples of suitable dosage ranges can be found in the scientific literature and in the *Physicians Desk Reference.* Many examples of suitable dosage ranges for other compounds described herein are also found in public literature or can be identified using known procedures. These dosage ranges can be modified as desired to achieve a desired result.

### IV. Gene Therapy

Another aspect of the present invention is using *in vivo* gene therapy methods to deliver interferon in combination and/or alternation with the two or more anti-HBV agents, to treat HBV. Gene therapy methods relate to the introduction of nucleic acid (DNA, RNA, and antisense DNA or RNA) sequences into a host, such as an animal, and in particular a human, to increase the expression of the immunomodulator which may be operatively linked to a promoter and/or any other genetic elements necessary for its expression by the target tissue. Such gene therapy and delivery techniques and methods are known in the art, see, for example, WO 90/11092, WO 98/11779; U.S. Pat. No. 5,693,622, 5,705,151, 5,580,859; Tabata H. et al. (1997) Cardiovasc. Res. 35(3):470-479, Chao J et al. (1997) Pharmacol. Res. 35(6):517-522, Wolff J. A. (1997) Neuromuscul. Disord. 7(5):314-318, Schwartz B. et al. (1996) Gene Ther. 3(5):405-411, Tsurumi Y. et al. (1996) Circulation 94(12):3281-3290.

The polypeptide vector constructs used in the gene therapy method are preferably constructs that will not integrate into the host genome nor will they contain sequences that allow for replication. Any strong promoter known to those skilled in the art can be used for driving the expression of DNA.

The polypeptide constructs may be delivered by any method that delivers injectable materials to the cells of an animal, such as, injection into the interstitial space of tissues (heart, muscle, skin, lung, liver, intestine and the like). The polypeptide constructs can be delivered in a pharmaceutically acceptable liquid or aqueous carrier.

The polypeptide construct can be delivered to the interstitial space of tissues within an animal, including the muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts.

In a further embodiment of the invention, cells that are genetically engineered to express the immunomodulator are administered to a patient in vivo. Such cells may be obtained from the patient or an MHC compatible donor and can include, but are not limited to fibroblasts, bone marrow cells, blood cells (e.g., lymphocytes), adipocytes, muscle cells, endothelial cells etc. The cells are genetically engineered in vitro using recombinant DNA techniques to introduce the coding sequence of polypeptides of the immunomodulator, or alternatively, by transduction (using viral vectors, and preferably vectors that integrate the transgene into the cell genome) or transfection procedures, including, but not limited to, the use of plasmids, cosmids, YACs, naked DNA, electroporation, liposomes, etc. The coding sequence of the immunomodulator can be placed under the control of a strong constitutive or inducible promoter or promoter/enhancer to achieve immunomodulator expression, and preferably secretion. The engineered cells which express and preferably secrete the immunomodulator can be introduced into the patient systemically, e.g., in the circulation, or intraperitoneally.

Alternatively, the cells can be incorporated into a matrix and implanted in the body, e.g., genetically engineered endothelial cells can be implanted as part of a lymphatic or vascular graft. (See, for example, Anderson et al. U.S. Pat. No. 5,399,349; and Mulligan & Wilson, U.S. Pat. No. 5,460,959).

When the cells to be administered are non-autologous or non-MHC compatible cells, they can be administered using well known techniques which prevent the development of a host immune response against the introduced cells. For example, the cells may be introduced in an encapsulated form which, while allowing for an exchange of components with the immediate extracellular environment, does not allow the introduced cells to be recognized by the host immune system.

Eukaryotic cells that may be transduced with the infectious viral particles containing a nucleic acid, gene or gene fragment thereof for the expression of an immunomodulator include, but are not limited to, primary cells, such as primary nucleated blood cells, such as leukocytes, granulocytes, monocytes, macrophages, lymphocytes (including T-lymphocytes and B-lmphocytes), totipotent stem cells, and tumor infiltrating lymphocytes (TIL cells); bone marrow cells; endothelial cells; epithelial cells; keratinocytes; stem cells; hepatocytes, including hepatocyte precursor cells; hepatocytes, including hepatocyte precursor cells; fibroblasts; mesenchymal cells; mesothelial cells; and parenchymal cells.

In one embodiment, the cells may be targeted to a specific site, whereby the cells function as a therapeutic at such site. Alternatively, the cells may be cells that are not targeted to a specific site, and such cells function as a systemic therapeutic.

Transduced cells may be used, for example, in the treatment of HBV by introducing to host cells, such as blood cells that have been removed from a patient and expanded in culture, infectious viral particles in accordance with the present invention which contain genes that encode an immunomodulator. The cells can be expanded in number before or after transduction with the infectious viral particles containing the desired genes. Thus, the procedure is performed in such a manner that upon injection into the patient, the transformed cells will produce an immunomodulator in the patient's body.

The gene or nucleic acid carried by the transduced cells specifically comprises the sequence for an interferon but can be also comprise any sequence that directly or indirectly enhances the therapeutic effects of the cells. The gene carried by the transduced cells can also include sequences that allow the transduced cells to exert a therapeutic effect that it would not ordinarily have, such as a gene encoding a clotting factor useful in the treatment of hemophilia. The gene can encode one or more products having therapeutic effects. Suitable genes or nucleic acids encode, interferons (eg. alpha., beta, gamma.-interferons),

It is typical but not required to deliver the nucleic acid to the cell using a carrier known as a "vector." The most common types of vectors used in gene therapy are viruses. Scientists use viruses because they have a unique ability to enter a cell's DNA. Viruses used as vectors in gene therapy are genetically disabled; they are unable to reproduce themselves. Most gene therapy clinical trials rely on mouse retroviruses to deliver the desired gene. Other viruses used as vectors include adenoviruses, adeno-associated viruses, poxviruses and the herpes virus.

For example, cells from the patient are removed and grown in the laboratory. The cells are exposed to the virus that is carrying the desired gene. The virus enters the cells, and the desired gene becomes part of the cells' DNA. The cells grow in the laboratory and are then returned to the patient. This type of gene therapy is called *ex vivo,* which means "outside the body." The gene is transferred into the patient's cells while the cells are outside the patient's body. In other studies, vectors or liposomes (fatty particles) are used to deliver the desired gene to cells in the patient's body. This form of gene therapy is called *in vivo,* because the gene is transferred to cells inside the patient's body.

When viral vectors are used to carry genes into the body, they might alter more than the intended cells. Another danger is that the new gene might be inserted in the wrong location in the DNA, possibly causing cancer or other damage. In addition, when DNA is injected directly, or when a liposome delivery system is used, there is a chance that the DNA could be introduced into reproductive cells, producing inheritable changes.

Other concerns include the possibility that transferred genes could be "overexpressed," producing so much of the missing protein as to be harmful; that the viral vector could cause inflammation or an immune reaction; and that the virus could be transmitted from the patient to other individuals or into the environment.

There are many vectors known in the art. Any known vector can be used in the present invention. In a preferred embodiment of the present invention, the vector can target a specific cell type for specific gene delivery.

### Adenoviral Vectors

Any of the adenoviral vectors can be used to transfect cells and/or cell lines to express and/or secrete an interferon. Adenoviruses are non-enveloped viruses containing a linear double stranded DNA genome. While there are over 40 serotype strains of adenovirus, most of which cause benign respiratory tract infections in humans, subgroup C serotypes 2 or 5 are predominantly used as vectors. The life cycle does not normally involve integration into the host genome, rather they replicate as episomal elements in the nucleus of the host cell and consequently there is no risk of insertional mutagenesis. The wild type adenovirus genome is approximately 35 kb of which up to 30 kb can be replaced with foreign DNA (Smith, 1995, Verma and Somia, 1997). There are four early transcriptional units (E1, E2, E3 and E4) that have regulatory functions, and a late transcript, which codes for structural proteins. Progenitor vectors have either the E1 or E3 gene inactivated, with the missing gene being supplied in trans either by a helper virus, plasmid or integrated into a helper cell genome (human fetal kidney cells, line 293, Graham, F. L., Smiley, J., Russell, W. L. and Nairn, R. (1997). General Virology 36: 59-72.). Second generation vectors additionally use an E2a temperature sensitive mutant (Engelhardt, J. F., Litsky, L., and Wilson, J. M. (1994). Human Gene Therapy 5: 1217-1229.) or an E4 deletion (Armentano, D., Zabner, J., et al. (1997). Journal of Virology 71: 2408-2416.). The most recent "gutless" vectors contain only the inverted terminal repeats (ITRs) and a packaging sequence around the transgene, all the necessary viral genes being provided in trans by a helper virus (Chen, H., Mack, L. M., Kelly, R., et al. (1997). Proceedings of the National Academy of Sciences of the U.S.A. 94: 1645-1650.).

Adenoviral vectors are very efficient at transducing target cells in vitro and vivo, and can be produced at high titres (>10¹¹/mL). With the exception of Geddes, B. J., Harding, T. C., Lightman, S. L. and Uney, J. B. (1997). Nature Medicine 3: 1402-1404.), who showed prolonged transgene expression in rat brains using an E1 deletion vector, transgene expression in vivo from progenitor vectors tends to be transient (Verma, I. M. and Somia, N. (1997). Gene therapy - promises, problems and prospects. Nature 389: 239-242.). Following intravenous injection, 90% of the administered vector is degraded in the liver by a non-immune mediated mechanism (Worgall, S., Wolff, G., Falck-Pedersen, E. and Crystal R. G. (1997). Human Gene Therapy 8: 37-44.). Thereafter, an MHC class I restricted immune response occurs, using CD8+ CTLs to eliminate virus infected cells and CD4+ cells to secrete IFN-alpha which results in anti-adenoviral antibody (Yang, Y. and Wilson, J. M. (1995). Journal of Immmunology 155: 2564-2569.). Alteration of the adenoviral vector can remove some CTL epitopes, however the epitopes recognized differ with the host MHC haplotype (Sparer, T. E., Wynn, S. G., Clarket al. (1997). Journal of Virology 71: 2277-2284. Jooss, K., Ertl, H. C. J. and Wilson, J. M. (1998). Journal of Virology 72: 2945-2954.). The remaining vectors, in those cells that are not destroyed, have their promoter inactivated (Armentano, D., Zabner, J., et al. (1997). Journal of Virology 71: 2408-2416.) and persisting antibody prevents subsequent administration of the vector.

Approaches to avoid the immune response involving transient immunosupressive therapies have been successful in prolonging transgene expression and achieving secondary gene transfer (Jooss, K., Yang, Y. and Wilson, J. M. (1996). Human Gene Therapy 7: 1555-1566. Kay, M. A., Meuse, L., et al. (1997). Proceedings of the National Academy of Sciences of the U.S.A. 94: 4686-4691). A less interventionist method has been to induce oral tolerance by feeding the host UV inactivated vector (Kagami, H., Atkinson, J. C., et al. (1998). Human Gene Therapy 9: 305-313.). However, it is desirable to manipulate the vector rather than the host. Although only replication deficient vectors are used, viral proteins are expressed at a very low level which are presented to the immune system. The development of vectors containing fewer genes, culminating in the "gutless" vectors which contain no viral coding sequences, has resulted in prolonged in vivo transgene expression in liver tissue (Schiedner, G., Morral, N., et al. (1998). Nature Genetics 18: 180-183.). The initial delivery of large amounts of DNA packaged within adenovirus proteins, the majority of which will be degraded and presented to the immune system may still cause problems for clinical trials. Moreover the human population is heterogeneous with respect to MHC haplotype and a proportion of the population will have been already exposed to the adenoviral strain (Gahry-Sdard, H., Molinier-Frenkel, V., et al. (1997). Journal of Clinical Investigation 100: 2218-2226.)

Until recently, the mechanism by which the adenovirus targeted the host cell was poorly understood. Tissue specific expression was therefore only possible by using cellular promoter/enhancers e.g. the myosin light chain 1 promoter (Shi, Q., Wang, Y. and Worton, R. (1997). Human Gene Therapy 8: 403-410.) and the smooth muscle cell SM22a promoter (Kim, S., Lin, H., et al. (1997). Journal of Clinical Investigation 100: 1006-1014.), or by direct delivery to a local area (Rome, J. J., Shayani, V., et al. (1994). Human Gene Therapy 5: 1249-1258.). Uptake of the adenovirus particle has been shown to be a two stage process involving an initial interaction of a fibre coat protein in the adenovirus with a cellular receptor or receptors, which include the MHC class I molecule (Hong, S. S., Karayan, L., et al. (1997). EMBO Journal 16: 2294-2306.) and the coxsackievirus-adenovirus receptor (Bergelson, J. M., Cunningham J. A., et al. (1997). Science 275: 1320-1323.). The penton base protein of the adenovirus particle then binds to the integrin family of cell surface heterodimers (Wickham, T. J., Mathias, P., et al. (1993). Cell 73: 309-319.) allowing internalisation via receptor mediated endocytosis. Most cells express primary receptors for the adenovirus fibre coat protein, however internalisation is more selective (Harris, J. D. and Lemoine, N. R. (1996). Trends in Genetics 12: 400-404.). Methods of increasing viral uptake include stimulating the target cells to express an appropriate integrin (Davison, E., Diaz, R. M., et al. (1997). Journal of Virology 71: 6204-6207.) and conjugating an antibody with specificity for the target cell type to the adenovirus (Wickham, T. J., Lee, G. M, et al. (1997b). Journal of Virology 71: 7663-7669. Goldman, C. K., Rogers, B. E., et al. (1997). Cancer Research 57: 1447-1451.). The use of antibodies though increases the production difficulties of the vector and the potential risk of activating the complement system. By incorporating receptor binding motifs into the fibre coat protein, Wickham *et al.* (Wickham, T. J., Tzeng, E., et al. (1997a). Journal of Virology 71: 8221-8229.) were able to redirect the virus to bind the integrin expressed by damaged endothelial or smooth muscle cells, or heparin sulphate receptors which is expressed by numerous cells types.

Any of the adeno-associated viral vectors can be used to transfect cells and/or cell lines to express and/or secrete an immunomodulator. Adeno-associated viruses (AAV) are non-pathogenic human parvoviruses, dependant on a helper virus, usually adenovirus, to proliferate. They are capable of infecting both dividing and non dividing cells, and in the absence of a helper virus integrate into a specific point of the host genome (19q 13-qter) at a high frequency (Kotin, R. M., Siniscalco, M., et al. (1990). Proceedings of the National Academy of Sciences of the U.S.A. 87: 2211-2215.). The wild type genome is a single stranded DNA molecule, consisting of two genes; rep, coding for proteins which control viral replication, structural gene expression and integration into the host genome, and cap, which codes for capsid structural proteins. At either end of the genome is a 145 bp terminal repeat (TR), containing a promoter.

When used as a vector, the rep and cap genes are replaced by the transgene and its associated regulatory sequences. The total length of the insert cannot greatly exceed 4.7 kb, the length of the wild type genome (Smith, A. E. (1995). Annual Review of Microbiology 49: 807-838.). Production of the recombinant vector requires that rep and cap are provided in trans, along with helper virus gene products (Ela, E1b, E2a, E4 and VA RNA from the adenovirus genome). The conventional method is to cotransfect two plasmids, one for the vector and another for rep and cap, into 293 cells infected with adenovirus (Samulski, R. J., Chang, L., and Shenk, T. (1989). Journal of Virology 63: 3822-3828.). This method, however, is cumbersome, low yielding (<10⁴ particles/ml) and prone to contamination with adenovirus and wild type AAV. One of the reasons for the low yield is the inhibitory effect of the rep gene product on adenovirus replication (Vincent, K. A, Piraino, S. T. and Wadsworth, S. C. (1997). Journal of Virology 71: 1897-1905.). More recent protocols remove all adenoviral structural genes and use rep resistant plasmids (Xiao, X., Li, J. and Samulski, R. J. (1998) Journal of Virology 72: 2224-2232.) or conjugate a rep expression plasmid to the mature virus prior to infection (Fisher, K. J., Kelley, W. M, Burda, J. F. and Wilson, J. M. (1996) Human Gene Therapy 7: 2079-2087.).

In the absence of rep, the AAV vector will only integrate at random, as a single provirus or head to tail concatamers, once the terminal repeats have been slightly degraded (Rutledge, E. A. and Russell, D. W. (1997). Journal of Virology 71: 8429-8436.). Interest in AAV vectors has been due to their integration into the host genome allowing prolonged transgene expression. Gene transfer into vascular epithelial cells (Maeda, Y., Ikeda, U., et al. (1997). Cardiovascular Research 35: 514-521.), striated muscle (Fisher, K. J., Jooss, K., et al. (1997). Nature Medicine 3: 306-316. Herzog, R. W., et al. (1997). Proceedings of the National Academy of Sciences of the U.S.A. 94: 5804-5809.) and hepatic cells (Snyder, R. O., Miao, C. H., et al. (1997). Nature Genetics 16: 270-275.) has been reported, with prolonged expression when the transgene is not derived from a different species. Neutralising antibody to the AAV capsid may be detectable, but does not prevent readministration of the vector or shut down promoter activity. It is possibly due to the simplicity of the viral capsid, that the immune response is so muted. As AAV antibodies will be present in the human population this will require further investigation. There has been no attempt to target particular cell types other than by localised vector delivery.

In particular, the adeno-associated vectors disclosed in U.S. Patent No. 5,693,531, which is hereby incorporated by reference, can be used, including AAVp5neo; pSV-β-Galactosidase; TRF169; LZ11; pSP72; pSP72nLacZ; pAdRSV4; pAdRSVnLacZ; AAVrnLac; SV40; pBluescriptSK; pSV40 ori AAV 1; and pKMT11.

### Retroviral Vectors

Any of the retroviral vectors can be used to transfect cells and/or cell lines to express and/or secrete an immunomodulator. Retroviruses are a class of enveloped viruses containing a single stranded RNA molecule as the genome. Following infection, the viral genome is reverse transcribed into double stranded DNA, which integrates into the host genome and is expressed as proteins. The viral genome is approximately 10kb, containing at least three genes: gag (coding for core proteins), pol (coding for reverse transcriptase) and env (coding for the viral envelope protein). At each end of the genome are long terminal repeats (LTRs) which include promoter/enhancer regions and sequences involved with integration. In addition there are sequences required for packaging the viral DNA (psi) and RNA splice sites in the env gene. Some retroviruses contain protooncogenes, which when mutated can cause cancers, however, in the production of vectors these are removed. Retroviruses can also transform cells by integrating near to a cellular protooncogene and driving inappropriate expression from the LTR, or by disrupting a tumor suppresser gene. This event, termed insertional mutagenesis, though extremely rare could still occur when retroviruses are used as vectors.

Retroviral vectors are most frequently based upon the Moloney murine leukemia virus (Mo-MLV), which is an amphotrophic virus, capable of infecting both mouse cells, enabling vector development in mouse models, and human cells, enabling human treatment. The viral genes (gag, pol and env) are replaced with the transgene of interest and expressed on plasmids in the packaging cell line. Because the non-essential genes lack the packaging sequence (psi) they are not included in the virion particle. To prevent recombination resulting in replication competent retroviruses, all regions of homology with the vector backbone should be removed and the non-essential genes should be expressed by at least two transcriptional units (Markowitz, D., Goff, S. and Bank, A. (1988). A safe packaging line for gene transfer: separating viral genes on two different plasmids. Journal of Virology 62: 1120-1124.). Even so, replication competent retroviruses do occur at a low frequency.

The essential regions include the 5' and 3' LTRs, and the packaging sequence lying downstream of the 5' LTR. Transgene expression can either be driven by the promoter/ enhancer region in the 5' LTR, or by alternative viral (e.g. cytomegalovirus, Rous sarcoma virus) or cellular (e.g. beta actin, tyrosine) promoters. Mutational analysis has shown that up to the entire gag coding sequence and the immediate upstream region can be removed without effecting viral packaging or transgene expression (Kim, S. H., Yu, S. S., et al. (1998). Journal of Virology 72: 994-1004.). However the exact positioning of the transgene start codon and small alterations of the 5' LTR influence transgene expression (Rivire, I., Brose, K. and Mulligan, R. C. (1995). Proceedings of the National Academy of Sciences of the U.S.A. 92: 6733-6737.). To aid identification of transformed cells selectable markers, such as neomycin and beta galactosidase, can be included and transgenes expression can be improved with the addition of internal ribosome sites (Saleh, M. (1997). Human Gene Therapy 8: 979-983.). The available carrying capacity for retroviral vectors is approximately 7.5 kb (Verma, I. M. and Somia, N. (1997). Nature 389: 239-242.), which is too small for some genes even if the cDNA is used.

The retroviral envelope interacts with a specific cellular protein to determine the target cell range. Altering the env gene or its product has proved a successful means of manipulating the cell range. Approaches have included direct modifications of the binding site between the envelope protein and the cellular receptor, however these approaches tend to interfere with subsequent internalisation of the viral particle (Harris, J. D. and Lemoine, N. R. (1996). Trends in Genetics 12: 400-404.). By replacing a portion of the env gene with 150 codons from the erythropoietin protein (EPO), Kasahara et al. (Kasahara, N., Dozy, A. M. and Kan, Y. W. (1994). Science 266: 1374-1376.) were able to target EPO receptor bearing cells with high affinity. Coupling an antibody to the viral particle with affinity for a second cell specific antibody via a streptovadin bridge, improves viral uptake, but internalisation tends to lead to viral degradation (Roux, P., Jeanteur, P., and Piechaczyk, M. (1989). Proceedings of the National Academy of Sciences USA 86: 9079-9083.). Neda et al (Neda, H., Wu, C. H., and Wu. G. Y. (1991) The Journal of Biological Chemistry 266: 14143-14146.) treated viral particles with lactose which resulted in uptake by cells, principally hepatocytes, expressing asiaglycoprotein receptors. Subsequently there was efficient viral transgene expression, possibly due to acidification of the endosome allowing fusion of the viral envelope with the endosome membrane.

Viruses differ with respect to their tropisms, therefore by replacing the env gene with that of another virus, the host range can be extended, in a technique known as pseudotyping. Vesicular stomatitis virus G protein has been included in Mo-MLV derived vectors (Burns, J. C., Matsubara, T., et al. (1994). Developmental Biology 165: 285-289.), which are also more stable when purified by ultracentrifugation. Recently, Qing (Qing, K., Bachelot, T., Mukherjee, P., et al. (1997). Journal of Virology 71: 5663-5667.) improved transduction into numerous cell lines by first treating the recipient cells with an adeno-associated vector (discussed below) expressing the cellular receptor for retroviral envelope protein.

A requirement for retroviral integration and expression of viral genes is that the target cells should be dividing. This limits gene therapy to proliferating cells in vivo or ex vivo, whereby cells are removed from the body, treated to stimulate replication and then transduced with the retroviral vector, before being returned to the patient. Ex vivo cells can be more efficiently transduced, due to exposure to higher virus titres and growth factors (Glimm, H., Kiem, H. P., et al. (1997). Human Gene Therapy 8: 2079-2086.). Furthermore ex vivo treated tumor cells will associate with the tumor mass and can direct tumoricidal effects (Oldfield, E. H. and Ram, Z. (1995). Human Gene Therapy 6: 55-85.; Abdel-Wahab, Z., Weltz, C., et al. (1997). Cancer 80: 401-412.).

Lentiviruses are a subclass of retroviruses that are able to infect both proliferating and non-proliferating cells. They are considerably more complicated than simple retroviruses, containing an additional six proteins, tat, rev, vpr, vpu, nef and vif. Current packaging cell lines have separate plasmids for a pseudotype env gene, a transgene construct, and a packaging construct supplying the structural and regulatory genes in trans (Naldini, L., Blmer, U., et al. (1996). Science 272: 263-267.). Early results using marker genes have been promising, showing prolonged in vivo expression in muscle, liver and neuronal tissue (Blmer, U., Naldini, L., et al. (1997). Journal of Virology 71: 6641-6649. ; Miyoshi, H., Takahashi, M., Gage, F. H. and Verma, I. M. (1997). Proceedings of the National Academy of Sciences of the U.S.A. 94: 10319-10323. ; Kafri, T., Blmer, U., et al. (1997). Nature Genetics 17: 314-317). Interestingly the transgenes are driven by an internally engineered cytomegalovirus promoter, which unlike when in MoMLV vectors, is not inactivated. This may be due to the limited inflammatory response to the vector injection, which was equal in magnitude to the saline control (Blmer, U., Naldini, L., Kafri, T., Trono, D., Verma, I. M. and Gage, F. H. (1997). Journal of Virology 71: 6641-6649).

The lentiviral vectors used are derived from the human immunodeficiency virus (HIV) and are being evaluated for safety, with a view to removing some of the non-essential regulatory genes. Mutants of vpr and vif are able to infect neurones with reduced efficiency, but not muscle or liver cells (Blmer, U., Naldini, L., Kafri, T., Trono, D., Verma, I. M. and Gage, F. H. (1997). Journal of Virology 71: 6641-6649; Kafri, T., Blmer, U., et al. (1997). Nature Genetics 17: 314-317.).

In a particular embodiment, the retroviral vectors pLXIN, pSIR, pLXSH, pLNCX, pLAPSN, pFB and pFB-Neo are used.

### Herpes Simplex Viral Vectors

Any of the herpes simplex viral vectors can be used to transfect cells and/or cell lines to express and/or secrete an immunomodulator. Herpes simplex virus type 1 (HSV-1) is a human neurotropic virus, consequently interest has largely focused on using HSV-1 as a vector for gene transfer to the nervous system. The wild type HSV-1 virus is able to infect neurones and either proceed into a lytic life cycle or persist as an intranuclear episome in a latent state. Latently infected neurones function normally and are not rejected by the immune system. Though the latent virus is transcriptionally almost silent, it does possess neurone specific promoters that are capable of functioning during latency. Antibodies to HSV-1 are common in the human population, however complications due to herpes infection, such as encephalitis, are very rare.

The viral genome is a linear double stranded DNA molecule of 152 kb. There are two unique regions, long and short (termed UL and US) which are linked in either orientation by internal repeat sequences (IRL and IRS). At the non-linker end of the unique regions are terminal repeats (TRL and TRS). There are up to 81 genes (Marconi, P., Krisky, D., et al. (1996). Proceedings of the National Academy of Sciences USA 93: 11319-11320.), of which about half are not essential for growth in cell culture. Once these non essential genes have been deleted, 40-50 kb of foreign DNA can be accommodated within the virus (Glorioso, J. C., DeLuca, N. A. and Fink, D. J (1995). Annual Review of Microbiology 49: 675-710.). Three main classes of HSV-1 genes have been identified, namely the immediate-early (IE or alpha) genes, early (E or beta) genes and late (L or gamma) genes.

Following infection in susceptible cells, lytic replication is regulated by a temporally co-ordinated sequence of gene transcription. Vmw65 (a tegument structural protein) activates the immediate early genes (IP0, ICP4, ICP22, ICP27 and ICP477) that are transactivating factors allowing the production of early genes. The early genes encode genes for nucleotide metabolism and DNA replication. Late genes are activated by the early genes and code for structural proteins. The entire cycle takes less than 10h and invariably results in cell death.

The molecular events leading to the establishment of latency have not been fully determined. Gene expression during latency is driven by the latency associated transcripts (LATs) located in the IRL region of the genome. Two LATs (2.0 and 1.5kb) are transcribed in the opposite direction to the IE gene ICP0. LATs have a role in HSV-1 reactivation from latency (Steiner, I., Spivack, J. G., et al. (1989). EMBO Journal 8: 505-511.) and the establishment of latency (Sawtell, N. M. and Thompson, R. L. (1992). Journal of Virology 66: 2157-2169.). Two latency active promoters that drive expression of the LATs have been identified (Marconi, P., Krisky, D., et al.. (1996). Proceedings of the National Academy of Sciences USA 93: 11319-11320.) and may prove useful for vector transgene expression.

Two basic approaches have been used for production of HSV-1 vectors, namely amplicons and recombinant HSV-1 viruses. Amplicons are bacterially produced plasmids containing col E1 ori (an Escherishia coli origin of replication), OriS (the HSV-1 origin of replication), HSV-1 packaging sequence, the transgene under control of an immediate-early promoter and a selectable marker (Federoff, H. J., Geschwind, M. D., Geller, A. I. and Kessler, J. A. (1992). Proceedings of the National Academy of Sciences USA 89: 1636-1640.). The amplicon is transfected into a cell line containing a helper virus (a temperature sensitive mutant) which provides all the missing structural and regulatory genes in trans. Both the helper and amplicon containing viral particles are delivered to the recipient. More recent amplicons include an Epstein-Barr virus derived sequence for plasmid episomal maintenance (Wang, S. and Vos, J. (1996). Journal of Virology 70: 8422-8430.).

Recombinant viruses are made replication deficient by deletion of one the immediate-early genes e.g. ICP4, which is provided in trans. Though they are less pathogenic and can direct transgene expression in brain tissue, they are toxic to neurones in culture (Marconi, P., Krisky, D., et al.. (1996). Proceedings of the National Academy of Sciences USA 93: 11319-11320.). Deletion of a number of immediate-early genes substantially reduces cytotoxicity and also allows expression from promoters that would be silenced in the wild type latent virus. These promoters may be of use in directing long term gene expression.

Replication-conditional mutants are only able to replicate in certain cell lines. Permissive cell lines are all proliferating and supply a cellular enzyme to complement for a viral deficiency. Mutants include thymidine kinase (During, M. J., Naegele, J. R., OMalley, K. L. and Geller, A. I. (1994). Science 266: 1399-1403.), ribonuclease reductase (Kramm, C. M., Chase, M., et al. (1997). Human Gene Therapy 8: 2057-2068.), UTPase, or the neurovirulence factor g34.5 (Kesari, S., Randazzo, B. P., et al. (1995). Laboratory Investigation 73: 636-648.).

### Non-viral Vectors

Viral vectors all induce an immunological response to some degree and may have safety risks (such as insertional mutagenesis and toxicity problems). Further, their capacity is limited and large scale production may be difficult to achieve. Therefore, in one embodiment of the invention, non-viral methods of gene transfer are used, which may require only a small number of proteins, have a virtually infinite capacity, have no infectious or mutagenic capability and large scale production is possible using pharmaceutical techniques. There are three methods of non-viral DNA transfer, namely: naked DNA, liposomes and molecular conjugates.

### Naked Nucleic Acids

Naked DNA or nucleic acids can be used to deliver the interferon to the host. It can be delivered, for example, in the form of a plasmid can be directly injected into muscle cells (Wolff, J. A., Malone, R. W., Williams, P., Chong, W., Acsadi, G., Jani, A. and Felgner P. L. (1990). Science 247: 1465-1468.) or attached to gold particles that are bombarded into the tissue (Cheng, L., Ziegelhoffer, P. R. and Yang, N. S. (1993). Proceedings of the National Academy of Sciences of the U.S.A. 90: 4455-4459.). The terms "naked" nucleic acid, DNA or RNA refer to sequences that are free from any delivery vehicle that act to assist, promote, or facilitate entry into the cell, including viral sequences, viral particles, liposome formulations, lipofectin or precipitating agents and the like. Though not very efficient, this can result in prolonged low level expression in vivo. Unlike other gene therapies techniques, one major advantage of introducing naked nucleic acid sequences into target cells is the transitory nature of the immunomodulator synthesis in the cells. Studies have shown that non-replicating DNA sequences can be introduced into cells to provide production of the desired immunomodulator for periods of up to six months. The simplicity of this method, and sustained expression has led to the development of DNA vaccines. Compared to conventional attenuated and protein based vaccines, they are unaffected by pre-existing immunity e.g. due to maternal antibodies, relatively cheap, and can deliver a number of pathogen antigens on a single plasmid (Manickan, E., Karem, K. L., and Rouse, B. T. (1997). Critical Reviews in Immunology 17: 139-154.). DNA vaccines are being developed for those pathogens where there is no existing vaccine e.g. HIV (Lekutis, C., Shiver, J. W., Liu, M. A., and Letvin, L. N. (1997). The Journal of Immunology 158: 4471-4477.) or the current vaccine not fully effective e.g. influenza (Macklin, M. D., McCabe, D., et al. (1998). Journal of Virology 72: 1491-1496.). By using a highly conserved gene Ulmer et al. (Ulmer, J. B., Donnelly, J. J., et al. (1993). Science 254: 1745-1749.) were able to immunize mice against two serologically distinct influenza virus strains. In most cases however, DNA vaccines have not been shown to be better than the existing vaccines (Macklin, M. D., McCabe, D., et al. (1998). Journal of Virology 72: 1491-1496.). The actual type of immune response can be controlled by cotransformation of a gene coding for the appropriate cytokine (Xiang, Z. and Ertl, H. C. (1995). Immunity 2: 129-135.) and this method may prove useful in redirecting inappropriate immune responses (Manickan, E., Karem, K. L., and Rouse, B. T. (1997). Critical Reviews in Immunology 17: 139-154.). Other uses for naked DNA include cancer immunopotentiation (discussed below, Corr. M., Tighe, H., Lee. D., Dudler, J., et al. (1997). The Journal of Laboratory Investigation 159: 4999-5004.), repair of pancreatic insulin function (Goldfine. I. D., German, M. S., Tseng, H., et al. (1997). Nature Biotechnology 15: 1378-1382.), and stimulation of collateral blood vessel development (Takeshita, S., Tsurumi, Y., et al. (1996). Laboratory Investigation 75: 487-501.). Expression of the gene product in muscle tissue can be improved by the coadministration of collagenase, papaverine and ischaemic conditions (Budker, V., Zhang, G., Danko, I., Williams, P. and Wolff, J. (1998). Gene Therapy 5: 272-276.). The use of a muscle specific promoter (Skarli, M., Kiri, A., et al. (1998). Gene Therapy 5: 514-520.) and other intragene regulatory sequences, such as the poly A and transcription termination sequence (Hartikka, J., Sawdey, M., et al. (1996). Human Gene Therapy 7: 1205-1217.) will also improve transgene expression.

For the naked polypeptide injection, an effective dosage amount of DNA or RNA will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The route of administration is by the parenteral route of injection into the interstitial space of tissues, or other parenteral routes may also be used, such as, inhalation of an aerosol formulation particularly for delivery to lungs or bronchial tissues, throat or mucous membranes of the nose. In addition, naked polypeptide constructs can be delivered to arteries during angioplasty by the catheter used in the procedure.

The interferon may also be delivered in liposome formulations (such as those taught in Felgner P. L. et al. (1995) Ann. NY Acad. Sci. 772:126-139 and Abdallah B. et al. (1995) Biol. Cell 85(1):1-7) which can be prepared by methods well known to those skilled in the art. Liposomes are lipid bilayers entrapping a fraction of aqueous fluid. DNA will spontaneously associate to the external surface of cationic liposomes (by virtue of its charge) and these liposomes will interact with the cell membrane (Felgner, J. H., Kumar, R., et al. (1994). Journal of Biological Chemistry 269: 2550-2561.). In vitro up to 90% of certain cell lines may be transfected. By including a small amount of an anionic lipid in an otherwise cationic liposome the DNA can be incorporated into the internal surface of the liposome, thus protecting it from enzymatic degradation. (Crespo et al, 1996, cited in Alio, S. F. (1997). Biochemical Pharmacology 54: 9-13.). To facilitate uptake into the cell as endosomes, targeting proteins have been included in liposomes, e.g. anti-MHC antibody (Wang, C., and Huang, L. (1987). Proceedings of the National Academy of Sciences USA 84: 7851-7855.) transferrin (Stavridis, J. C., Deliconstantinos, G., et al. (1986). Experimental Cell Research 164: 568-572.), and the Sendai virus or its F protein (Dzau, J. V., Mann, M. J, Morishita, R. and Kaneda, Y. (1996). Proceedings of the National Academy of Sciences USA 93: 11421-11425.). The Sendai virus additionally allows the plasmid DNA to escape from the endosome into the cytoplasm, thus avoiding degradation. The inclusion of a DNA binding protein (28 kDa high mobility group 1 protein) enhances transcription by bringing the plasmid into the nucleus (Dzau et al, 1997 Am J Cardiol. 1997 Nov 6;80(9A):33I-39I).

Molecular conjugates consist of protein or synthetic ligands to which a DNA binding agent has been attached. Delivery to the cell can be improved by using similar techniques to those for liposomes. Targeting proteins include asialoglycoprotein (Wagner, E., Cotten, M., Foisner, R. and Birnstiel, M. L. (1991). Proceedings of the National Academy of Sciences USA 88: 4255-4259.), transferrin (Wu, C. H., Wilson, J. M. and Wu., G. Y. (1989). Journal of Biological Chemistry. 264: 16985-16987.), polymeric IgA (Ferkol, T., Kaetzel, C. S. and Davis, P. B. (1993). Journal of Clinical Investigation 92: 2394-2400.) and adenovirus (Madon, J. and Blum, H. E. (1996). Hepatology 24: 474-481.). Transgene expression tends to be transient and is limited by endosome/lysosomal degradation.

Illustrative examples of the present invention are provided below.

### EXAMPLES

The abbreviations as used herein are meant to refer to the following:
- Ad IFN: Adenovirus vector expressing woodchuck interferon gamma
- Ad RFP: Adenovirus vector without woodchuck interferon gamma gene
- CCC DNA: Covalently closed circular viral form of DNA
- DNA: Deoxyribonucleic acid
- FTC: 5-fluoro-1-(2*R*,5*S*)-1,3-oxathiolan-5-yl]cytosine
- GFP: Green fluorescent protein
- HBV: Hepatitis B Virus
- IFN: Interferon
- L-FMAU: 1-(2-fluoro-5-methyl-β, L-arabinofuranosyl)thymine
- M1, M2: Month 1 or 2, respectively, after the beginning of the treatment
- PCNA: Proliferating Cell Nuclear Antigen
- PCR: Polymerase chain reaction
- Pfu: Plaque forming unit
- RFP: Red fluorescent protein
- RI: Replication intermediates
- RT: Reverse Transcription
- TUNEL: terminal deoxynucleotidyltransferase (TdT)-mediated dUTP nick-end labeling assay
- WHV: Woodchuck Hepatitis B Virus

### Example 1

### Administration of L-FMAU and β-L-FTC

In general, β-L-FTC was administrated following a protocol previously described by Cullen et al. (Cullen et al. Antimicrob. Agents Chemother. 1997, 41, 2076-2082). Specifically, β-L-FTC was injected intraperitoneally at 30mg/kg.

In experiments using L-FMAU, the compound was administered by intraperitoneal administration at a concentration described by Peek et al. (Peek et al. Hepatology 2001, 33, 254-66) and Zhu et al. (Zhu et al. J. Virol. 2001, 75, 311-22) (10 mg/kg).

### Animals

The woodchuck hepatitis B virus infection model represents a useful model for the evaluation of antivirals since it mimics the history of viral infection observed in humans with chronic hepatitis B. Several studies have shown that this animal model is reliable for use in the study of new nucleoside analogs, particularly in terms of antiviral efficacy and toxicity.

Twelve captive-born woodchucks *(Marmotta monax)* of approximately one year of age and chronically WHV infected were used for *in vivo* experiments. The treatment protocols were performed from July to December 2000.

When adenovirus was injected, blood samples were collected daily during the ten days after the adenovirus injection and twice a week thereafter. These samples were further evaluated for viral markers as described below.

### Interferon gene delivery system (Dr. Nassal, Freiburg, Germany)

The adenovirus vector was obtained according to procedures described by He et al. (He, T. C., et al. Proc Natl Acad Sci U.S.A. 1998, 95, 2509-14). The adenovirus vectors were replication defective due to deletions in the E1 and E3 region. A recombinant cassette with a coding cassette for the pre-IFN gamma gene (aa 1-143) under control of a CMV promoter associated with eCFP gene reporter under the control of SV40 promoter was inserted in the original E1 region (454-3500) (**Figure 1**). The active woodchuck IFN gamma production efficacy was assessed by protection from VSV infection of the woodchuck cell line WCH17.

The same adenovector containing only the pSV40-eCFP cassette was used as control. Adenoviral vectors were administered by intravenous injection to target the liver. The vectors were administered at concentrations of 3x10⁷ to 3x10¹¹ pfu/animal, diluted in phosphate buffered saline (PBS).

### Example 2

### Evaluation of L-FMA U + β-L-FTC + Ad IFN

Eighteen woodchucks *(Marmota monax),* experimentally infected with WHV were purchased from Northeastern Wildlife (South Plymouth, N.Y.) The woodchucks were chronically infected and were used for *in vivo* experiments.

Both β-L-FTC (30 mg/kg) and L-FMAU (10 mg/kg) were administrated intraperitoneally. Daily injections were performed during the first week, followed by injections every other day for the next 7 weeks.

A new recombinant cassette of the woodchuck interferon gamma (IFN) gene under the control of a CMV promoter, which was associated with an eRFPnuc gene reporter under the control of a SV40 promoter was inserted in the original E1 region of the adenoviral vector (Ad IFN). The same adenovirus vector containing pSV40-controlled eGFPnuc alone was used as control (AdGFP) (provided by Dr. Michael Nassal, University of Freiburg, Germany).

For these experiments, intravenous injections of recombinant adenoviral vector expressing woodchuck interferon gamma were performed twice (at week 4 and week 8) with 3x10¹⁰ pfu per animal. The viral inoculation was validated in a previous study presented at the HBV meeting held in Amherst, MA, USA, August 2001 (see **Figure 2**).

During therapy, blood samples were collected every two days, and every day for five days after each injection of Ad IFN. After drug withdrawal, blood samples were collected every two days until viral load began to increase, then once a week and, then twice a month.

### Example 3

### Liver biopsy

Surgical liver biopsy was performed after laparatomy under general anesthesia prior to therapy (TO), twice during treatment (M1, M2) and 4 months after drug withdrawal (M3). The on-treatment biopsies were made 4 days after each adenovirus injection. A part of each sample was stored at -80°C for viral DNA analysis. Another part was fixed in formalin and embedded in paraffin for liver histology and immunostaining. The last part was fixed in 1% osmium tetroxide for electron microscopy study (TO and M2). A macroscopic liver examination was also performed during surgery.

### Analysis of intrahepatic viral DNA synthesis

Intrahepatic viral DNA from infected woodchucks was extracted at the time of liver biopsy. After homogenization in 10mM Tris-HCl (pH 7.5), 10 mM EDTA, the liver samples were divided in two parts, one for isolation of total viral DNA (after proteinase K digestion, phenol-chloroform extraction followed by ethanol precipitation) and the other for isolation of non-protein bound covalently close circular (CCC) viral DNA (after SDS-KCl precipitation of protein bound DNA, phenol-chloroform extraction followed by ethanol precipitation). To normalize the amount of DAN in each sample used for Southern blot analysis, DNA concentration was determined by UV densitometric analysis and compared with previously quantified DNA after electrophoresis through agarose gel. Five micrograms of nucleic acids corresponding to total DNA or CCC DNA preparations were then subjected to electrophoresis through 1.2 % agarose gels, alkalin transfer by blotting to nylon membrane, and viral DNA was detected by a specific hybridization as described above.

### Analysis of liver histology

Formalin-fixed liver biopsy tissue sections were stained with hematoxylin-eosin-safran (HES) stain and examined under a light microscope. The degree of hepatocyte necrosis (acidophilic bodies), the level of inflammation of the portal tract and the intralobular space, and the fibrosis stage were semi quantitatively assessed by using the Metavir score *(*Hepatology. 1994;20(1 Pt 1):15-20). Liver biopsy sections were also assessed for steatosis, ductular proliferation, hepatocyte dysplasia, and hepatocellular carcinoma.

Liver histology examination revealed signs of acute exacerbation of hepatitis that were comparable in both groups of animals that received Ad IFN or Ad GFP vector while mild inflammatory activity was observed in the untreated animal.

Immunostaining studies performed on liver biopsy sections obtained prior to injection and D5 post injection with a polyclonal anti WHV antibody, showed a significant decrease of the number of infected hepatocytes in woodchucks A37 and A36.

### Example 4

### Analysis of viremia

### Dot Blot Assay

Viremia was assessed by quantitative detection of woodchuck hepatitis B virus (WHV) DNA. WHV DNA was extracted from serum using HighPure PCR template DNA extraction kit (Roche daignostics), and samples corresponding to 50µL of serum were spotted on membranes (Biodyne B, 0.45 µM, Merck Eurolab) with serial dilutions of a WHV DNA standard. After fixation procedure, filters were hybridized with ³²P. labeled full-length WHV DNA probe. A comparative analysis of phosphoImager scans (Amersham Pharmacia Biotech) was performed with the ImageQuant software. The detection limit of the assay was 6.10⁷ copies/mL (200 pg/mL).

### Endogenous Polymerase Assay (EPA)

The effect of Ad IFN on the synthesis of viral plus strand DNA was determined using WHV particles in an endogenous polymerase assay (EPA). For comparison, the DNA dependent DNA polymerase inhibitor, phosphonoformic acid (PFA) was used as control. The endogenous polymerase activity corresponds to the completion of viral plus strand DNA in mature particles containing relaxed circular DNA. WHV associated DNA polymerase activity was assayed by mixing 50µL of partially purified viral particles with 25µL of reaction buffer (Tris-HCl 50mM pH7.6, MgCl₂ 40mM, NH₄Cl 60mM, NP40 0.5%, β-mercaptoethanol 10mM) containing: (i) dATP, dCTP, dGTP (100µL each) and ³H-dTTP (0.17µM). enzyme assays were performed as described by Hantz et al. (Hantz O, et al. 1992 Virology. Sep;190(1):193-200). Briefly, enzyme reactions were incubated at 37°C for 3 hours and spotted on GF/C fiber glass filters (Whatman), and extensively washed by trichloroacetic acid 5%. Incorporation of ³H-dTTP was measured in a Beckman LS 6000SC scintillation counter. The limit of detection of this assay was 1,000 cpm/mL.

### Real time PCR assay (Light Cycler)

In cases where viral titers were too low to be easily detected by Dot blot hybridization, real time PCR techniques were utilized (see for e.g. Dandri et al. Hepatology 2000, 32, 139-46). Real time PCR has been known to detect as few as 10 copies of human HBV DNA. Therefore, to complete the analysis of viremia, real time PCR was used to detect WHV DNA below 6x10⁷ copies/ml (200 pg/ml). The same WHV DNA standard was used for both the dot blot and real time PCR assays. PCR assays on Dot blot positive sample provided similar quantitative result. The lower limit of detection of this method was 25 WHV copies/µL.

Preliminary results of kinetics of the viral clearance and rebound in 3 woodchucks, analyzed by real time PCR, are presented in **Figure 10**. Viral titer was shown to reach a low value of 0.320 pg/ml, representing an average of 9x10⁴copies/ml. This technique could also be applied to further animal samples to determine the efficacy of β-L-FTC alone or in combination with immunomodulators, such as Ad IFN.

### Example 5

### Inoculum titer determination

To determine the antiviral efficacy and tolerance of the adenoviral vector inoculum, 4 animals received intravenous Ad-IFN injection at concentrations of 3.10⁷ (A33), 3x10⁹ (A30) and 3x10¹¹ pfu (A32 and A34).

The highest titered inoculum (3.10¹¹ pfu) induced a marked drop of viral load, however all 2 animals died at day 2 and day 5 post injection (WHV DNA did drop between D0 and day of death: from 147 ng/ml to 70 ng/ml and from 1067 ng/ml to 265 ng/ml respectively). The 3x10⁹ pfu inoculum induced a transient decrease from 70 ng/ml to approximately 30 ng/ml for 6 days. The lowest inoculum did not induce a significant viremia decrease **(****Figure 3**).

### Example 6

### Efficacy study: evaluation of the antiviral effect of the Ad IFN vector

Five woodchucks (A35, A36, A37, A38, A42) received 3x10⁹ pfu of the complete Ad IFN vector. A control group consisting of two woodchucks (A39, A40) received 3x10⁹ pfu of the Ad GFP vector, and one woodchuck (A41) was untreated (**Figures 4** and 5).

In the Ad IFN treated group, a transient WHV DNA decrease was observed in 3 of 5 treated animals (A35 from 46 ng/ml to 20 ng/ml; A37 from 76 to 20 ng/ml and A42 from 20 to 5 ng/ml) after adenovirus inoculation. WHV DNA results were also assessed by EPA (**Figures 4** and **5**). Southern Blot Hybridization of intrahepatic WHV DNA showed a marked decrease of the viral DNA (from 16 to 76%, except A42) as well as CCC DNA levels (from 2 to 76%) (**Figure 6**).

### Example 7

### Evaluation of L-FMAU and β-L-FTC.

Results showed a significant inhibition of viremia which was reduced approximately by 4 to 5 log units, down to the limit of detection of the Dot blot assay (6.10⁷ copies/ml) in all animals treated by β-L-FTC, L-FMAU, and Ad IFN (**Figure 7**). However, controls (untreated, Ad GFP) or woodchucks treated with Ad IFN alone showed no significant variation in viremia (**Figures 8** and **9**).

Treatment induced reduction of viral load was remarkably rapid. Viremia decreased to undetectable levels (<6.10⁷/ml) in less than one to two weeks after the beginning of the therapy.

Viremia in the animals treated with β-L-FTC, L-FMAU, and Ad IFN remained suppressed for various lengths of time following withdrawal of treatment. Furthermore, the viral load in treated animals did not return to pretreatment levels. Viremia level of two woodchucks (A52 and A49) treated with L-FMAU, β-L-FTC, and Ad IFN were still under detectable level at week 28 (6 months after drug withdrawal).

Results of combination therapy with an immunomodulator showed a rapid and significant decrease of viremia level (average of 5 log₁₀ decrease within two weeks). This decrease is significant compared to results obtained with β-L-FTC and L-FMAU alone. In previous studies, viral titers in serum dropped about 56 and 1,000 fold respectively in the same time frame. This observation indicates that the antiviral activity of β-L-FTC and L-FMAU was improved when combined together with an immunomodulator. Moreover, this antiviral effect was prolonged over time with a delayed viral rebound. Viremia remained undetectable in dot blot assay (<6.10⁷copies/ml) in serum samples of two animals (A49 and A52) 6 to 7 months after drug withdrawal. This prolonged effect is consistent with decreases in CCC DNA levels (-69% and -75% respectively). The delay in the rebound of viral replication following the cessation of therapy may reflect the time required to re-establish CCC DNA pools through reinfection of new hepatocytes and/or reamplification of existing intracellular pools of CCC DNA in infected cells.

### Intrahepatic CCC DNA.

Southern blot analysis of intrahepatic viral DNA (**Figure 11**) demonstrated changes in the levels of intrahepatic WHV replication intermediates that were generally consistent with viremia patterns. A marked decrease in the level of viral DNA synthesis was observed in both β-L-FTC, L-FMAU treated groups, with 82 to 93% decrease in the level of intrahepatic viral DNA intermediates compared to the pretreatment of levels (**Figures 12** and **13**). While CCC DNA persisted in the liver at M1 and M2, other WHV DNA replicative intermediates decreased dramatically.

Moreover, CCC DNA was also reduced by 24 to 47% during L-FMAU/β-L-FTC bitherapy. However, the inhibition of WHV replication by L-FMAU/β-L-FTC administration was not followed by the clearance of intrahepatic viral CCC DNA, as demonstrated by southern blot analysis.

However in untreated woodchuck, total or CCC DNA increased over time by + 64% and + 77% respectively (**Figure 14**).

Compared to Cullen et al. (Cullen et al. Antimicrob. Agents Chemother. 1997, 41, 2076-2082), Zhu et al. (Zhu et al. J. Virol. 2001, 75, 311-22) and Peek et al. (Peek et al. Hepatology 2001, 33, 254-66) results, β-L-FTC combined with IFN and L-FMAU have a significant antiviral effect. Replicative intermediates decreased to undetectable levels in the liver one month after the beginning of the treatment. In contrast, the intrahepatic CCC DNA levels declined at a much lower rate than replicative DNA. Combination of β-L-FTC and L-FMAU decreased viral CCC DNA 40% (versus a decrease of 98% for the DNA replication intermediates). This effect could be due to the antiviral effect of the L-FMAU alone as it was demonstrated by Peek, et al. (Peek et al. Hepatology 2001, 33, 254-66), who showed an average 10-fold decrease in CCC DNA). However, after 8 to 9 months of a treatment with a single drug, L-FMAU, drug resistant strains of WHV emerged (Zhou, T., et al. J. Viron. 2000, 74, 11754-63). L-FMAU in combination to β-L-FTC may avoid emergence of drug resistant strains and allow long-term treatment that could deplete the residual pool of CCC DNA.

### Example 8

### Intrahepatic WHV DNA synthesis.

Unlike the results with the combination therapy, Ad IFN alone did not affect total viral and CCC DNA levels in the liver (+44% and -6% respectively compared to + 43% for total DNA of Ad GFP group) (**Figure 15**).

### Example 9

### Evaluation of hepatocytes transduction by the recombinant adenovirus vector

To determine the number of hepatocytes infected by adenovirus, immunostaining with commercial antibody directed against the Green Fluorescent Protein (GFP) was performed. However, fluorescence observed was not specific when compared with controls, and may have been due to natural fluorescence of hepatocytes or perhaps biliary pigments.

### Example 10

### Histology examination

Examination of liver biopsy sections of animals treated with β-L-FTC combination therapy revealed inflammation with lobular lesions in the liver. All woodchucks of the group treated with Ad IFN showed increased inflammatory activity (**Figure 15**). Maximal inflammatory activity in Metavir score was observed in six biopsies in this group. In comparison, the group treated with L-FMAU and β-L-FTC did not show similar profiles. Only three of the six woodchucks in this group showed an increase of the inflammatory activity. In control groups, Metavir score was stable over time, even in the group treated with Ad IFN alone.

### Example 11

### Analysis of viremia

Viremia could be analyzed using the dot blot assay for WHV DNA and quantified with a phosphoimager until viremia returns to pretreatment values.

If elimination of virus is to be confirmed, DNA extracted from serum under PCR conditions should be undetectable by the dot blot assay (viremia under 6.10⁷ copies/ml). Furthermore, the DNA must be analyzed by a real time PCR (Light Cycler) assay. In these examples, the real-time PCR method was adapted for WHV detection DNA using specific primers

### Example 12

### Analysis of intrahepatic viral DNA synthesis

Liver sections of the a biopsy taken at 5 months post treatment were analyzed by southern blot hybridization to analyze intrahepatic viral DNA synthesis.

### Example 13

### WHV polymerase gene sequencing

Genome sequence from circulating virions could be analyzed after PCR amplification of the PreS and the RT regions, to determine whether or not escape mutants had been selected by one of the antiviral protocols.

WHV DNA was amplified by PCR from serum collected either at the end of the treatment period or at the best point before the animal death. DNA from serum was amplified for 35 cycles (94°C for 1 min, 50°C for 1 min, and 72°C for 1 min) with Taq polymerase and a specific primer pair (5'-AGATTGGTTGGTGCACTTCT-3 (nucleotides 385 to 403) and 5'-ATTGTCAGTGCCCAACA-3' (nucleotides 1468 to 1461), corresponding to the B and C domains of the reverse transcriptase gene, with reference to previously published sequences. To confirm that no viral DNA is present, another primer pair was used for nested PCR in samples found to be negative in the first round of amplification, specifically 5'-GGATGTATCTGCGGCGTTT-3' (nucleotides 510 to 528) and 5'-CCCAAATCAAGAAAAACAGAACA-3' (nucleotides 953 to 931).

### Example 14

### Examination of liver mitochondria by electron microscopy

Liver biopsy sections were fixed in 1% osmium tetroxide in 150 mM sodium cacodylate HCI (pH 7.4), dehydrated in graded ethanol, and embedded with methanolic uranyl acetate and lead citrate, and will be analyzed with a transmission electron microscope 1200EX to rule out mitochrondrial toxicity of the combination β-L-FTC therapy.

### Example 15

### Visualisation of the GFP or RFP

The number of cells infected by the adenovirus vector could be monitored by direct observation of a five-micrometers-thick deparaffinized liver tissue sections by confocal microscopy using appropriate filters.

### Example 16

### Evaluation of the number of WHV-infected hepatocytes

The number of VHW infected cells could be monitored by immunostaining for surface and capsid proteins. To do this, five-micrometers-thick deparaffinized liver tissue sections were incubated overnight with rabbit serum containing polyclonal antibody directed against the WHV core or pre-S proteins. The sections were thereafter incubation with a biotinylated goat anti-rabbit immunoglobulin G. The antigen-antibody complex was revealed using streptadivin-horseradish peroxidase. Quantification of the number of infected hepatocytes expressing viral proteins was performed in representative areas of the liver sections.

### Example 17

### Analysis of hepatocytes turnover

The number of hepatocytes undergoing apoptosis could be determined using the TUNEL (terminal deoxynucleotidyl transferase-mediated dUTP nick end-labeling) method (Kerr, J.F.R., Wyllie, A.H. and Currie, A.R. 1972 Br. J. Cancer 26:239-257). The number of hepatocytes that progress in the cell cycle mitosis could be determined by immunostaining for PCNA.

## Claims

1. Use of:
β-L-FTC, an ether lipid prodrug thereof, or a nucleotide prodrug thereof optionally with an alkylation, acylation or other lipophilic modification of the hydroxyl group or of the mono, di or triphosphate;
L-FMAU, an ether lipid prodrug thereof, or a nucleotide prodrug thereof optionally with an alkylation, acylation or other lipophilic modification of a hydroxyl group or of the mono, di or triphosphate; and
interferon;
or their pharmaceutically acceptable salts, independently optionally in pharmaceutically acceptable carriers; in the manufacture of a medicament for combined or alternate administration, for the treatment or prophylaxis of a human infected with hepatitis B virus.

2. The use of claim 1, wherein the interferon is selected from the group consisting of interferon alpha, pegylated interferon alpha, interferon alpha-2a, interferon alpha-2b, interferon beta, interferon gamma, and consensus interferon.

3. The use of claim 2, wherein the interferon is interferon alpha.

4. The use of claim 2, wherein the interferon is interferon gamma.

5. The use of claim 2, wherein the interferon is interferon beta.

6. A pharmaceutical composition comprising:
β-L-FTC or a prodrug as defined in claim 1;
L-FMAU or a prodrug as defined in claim 1; and
interferon;
or their pharmaceutically acceptable salts, independently optionally in pharmaceutically acceptable carriers, for combined or alternate use, in the treatment or prophylaxis of a human infected with hepatitis B virus.

7. Use of β-L-FTC in the manufacture of a medicament, when used in combination or alternation with L-FMAU and interferon, for the treatment or prophylaxis of a human infected with hepatitis B virus.

8. Use of L-FMAU in the manufacture of a medicament, when used in combination or alternation with β-L-FTC and interferon, for the treatment or prophylaxis of a human infected with hepatitis B virus.

9. Use of interferon in the manufacture of a medicament, when used in combination or alternation with β-L-FTC and L-FMAU, for the treatment or prophylaxis of a human infected with hepatitis B virus.

10. β-L-FTC for use in the treatment or prophylaxis of a human infected with hepatitis B virus, wherein the β-L-FTC is used in combination or alternation with L-FMAU and interferon.

11. L-FMAU for use in the treatment or prophylaxis of a human infected with hepatitis B virus, wherein the L-FMAU is used in combination or alternation with β-L-FTC and interferon.

12. Interferon for use in the treatment or prophylaxis of a human infected with hepatitis B virus, wherein the interferon is used in combination or alternation with β-L-FTC and L-FMAU.

13. The use of any one of claims 1 and 7 to 9, wherein the interferon is delivered in the form of a gene or gene fragment that expresses the interferon protein.

14. The composition for use as claimed in claim 6, the β-L-FTC for use as claimed in claim 10, the L-FMAU for use as claimed in claim 11 or the interferon for use as claimed in claim 12, wherein the interferon is in the form of a gene or gene fragment that expresses the interferon protein.

## Patentansprüche

1. Verwendung von:
β-L-FTC, einem Ether-Lipid-Prodrug davon oder einem Nukleotid-Prodrug davon, gegebenenfalls mit einer Alkylierungs-, Acylierungs- oder einer anderen lipophilen Modifikation der Hydroxylgruppe oder des Mono-, Di- oder Triphosphates;
L-FMAU, einem Ether-Lipid-Prodrug davon oder einem Nukleotid-Prodrug davon, gegebenenfalls mit einer Alkylierungs-, Acylierungs- oder einer anderen lipophilen Modifikation einer Hydroxylgruppe oder des Mono-, Di- oder Triphosphates; und
Interferon;
oder ihre pharmazeutisch unbedenklichen Salze, unabhängig gegebenenfalls in pharmazeutisch unbedenklichen Trägern; bei der Herstellung eines Medikamentes zur kombinierten oder abwechselnden Verabreichung, bei der Behandlung oder Prophylaxe eines Menschen, der mit Hepatitis-B-Virus infiziert ist.

2. Verwendung nach Anspruch 1, wobei das Interferon aus der aus Alpha-Interferon, pegyliertem Alpha-Interferon, Alpha-2a-Interferon, Alpha-2b-Interferon, Beta-Interferon, Gamma-Interferon, und Konsensus-Interferon bestehenden Gruppe ausgewählt ist.

3. Verwendung nach Anspruch 2, wobei es sich bei dem Interferon um Alpha-Interferon handelt.

4. Verwendung nach Anspruch 2, wobei es sich bei dem Interferon um Gamma-Interferon handelt.

5. Verwendung nach Anspruch 2, wobei es sich bei dem Interferon um Beta-Interferon handelt.

6. Pharmazeutische Zusammensetzung, umfassend:
β-L-FTC oder ein Prodrug nach Anspruch 1;
L-FMAU oder ein Prodrug nach Anspruch 1;
Interferon;
oder ihre pharmazeutisch unbedenklichen Salze, unabhängig gegebenenfalls in pharmazeutisch unbedenklichen Trägern; zur kombinierten oder abwechselnden Verabreichung, bei der Behandlung oder Prophylaxe eines Menschen, der mit Hepatitis-B-Virus infiziert ist.

7. Verwendung von β-L-FTC bei der Herstellung eines Medikamentes, bei Verwendung in Kombination oder Abwechslung mit L-FMAU und Interferon, zur Behandlung oder Prophylaxe eines Menschen, der mit Hepatitis-B-Virus infiziert ist.

8. Verwendung von L-FMAU bei der Herstellung eines Medikamentes, bei Verwendung in Kombination oder Abwechslung mit β-L-FTC und Interferon, zur Behandlung oder Prophylaxe eines Menschen, der mit Hepatitis-B-Virus infiziert ist.

9. Verwendung von Interferon bei der Herstellung eines Medikamentes, bei Verwendung in Kombination oder Abwechslung mit β-L-FTC und L-FMAU, zur Behandlung oder Prophylaxe eines Menschen, der mit Hepatitis-B-Virus infiziert ist.

10. β-L-FTC zur Verwendung bei der Behandlung oder Prophylaxe eines Menschen, der mit dem Hepatitis-B-Virus infiziert ist, wobei das β-L-FTC in Kombination oder Abwechslung mit L-FMAU und Interferon verwendet wird.

11. L-FMAU zur Verwendung bei der Behandlung oder Prophylaxe eines Menschen, der mit dem Hepatitis-B-Virus infiziert ist, wobei das L-FMAU in Kombination oder Abwechslung mit β-L-FTC und Interferon verwendet wird.

12. Interferon zur Verwendung bei der Behandlung oder Prophylaxe eines Menschen, der mit dem Hepatitis-B-Virus infiziert ist, wobei das Interferon in Kombination oder Abwechslung mit β-L-FTC und L-FMAU verwendet wird.

13. Verwendung nach einem der Ansprüche 1 und 7 bis 9, wobei das Interferon in der Form eines Gens oder Genfragmentes abgegeben wird, das das Interferonprotein exprimiert.

14. Zusammensetzung zur Verwendung nach Anspruch 6, das β-L-FTC zur Verwendung nach Anspruch 10, das L-FMAU zur Verwendung nach Anspruch 11 oder das Interferon zur Verwendung nach Anspruch 12, wobei das Interferon in der Form eines Gens oder Genfragmentes vorliegt, das das Interferonprotein exprimiert.

## Revendications

1. Utilisation de :
un β-L-FTC, une prodrogue à lipide-éther de celui-ci ou une prodrogue nucléotidique de celui-ci, facultativement avec une alkylation, une acylation ou une autre modification lipophile du groupe hydroxyle ou bien du mono-, du di- ou du triphosphate ;
un L-FMAU, une prodrogue à lipide-éther de celui-ci ou une prodrogue nucléotidique de celui-ci, facultativement avec une alkylation, une acylation ou une autre modification lipophile d'un groupe hydroxyle ou bien du mono-, du di- ou du triphosphate ; et
un interféron ;
ou bien leurs sels acceptables d'un point de vue pharmaceutique, en option indépendamment dans des transporteurs acceptables d'un point de vue pharmaceutique ; dans la fabrication d'un médicament, pour une administration combinée ou alternée, destiné au traitement ou à la prophylaxie d'un être humain infecté par le virus de l'hépatite B.

2. Utilisation selon la revendication 1, dans laquelle l'interféron est choisi dans le groupe constitué par l'interféron alpha, l'interféron alpha pégylé, l'interféron alpha-2a, l'interféron alpha-2b, l'interféron bêta, l' interféron gamma, et l' interféron consensus.

3. Utilisation selon la revendication 2, dans laquelle l'interféron est un interféron alpha.

4. Utilisation selon la revendication 2, dans laquelle l'interféron est un interféron gamma.

5. Utilisation selon la revendication 2, dans laquelle l'interféron est un interféron bêta.

6. Composition pharmaceutique comprenant :
un β-L-FTC ou une prodrogue, tel que défini dans la revendication 1 ;
un L-FMAU ou une prodrogue, tel que défini dans la revendication 1 ;
un interféron ;
ou bien leurs sels acceptables d'un point de vue pharmaceutique, en option indépendamment dans des transporteurs acceptables d'un point de vue pharmaceutique, destinée à une utilisation combinée ou alternée dans le traitement ou la prophylaxie d'un être humain infecté par le virus de l'hépatite B.

7. Utilisation d'un β-L-FTC dans la fabrication d'un médicament, lorsqu'il est utilisé en combinaison ou en alternance avec du L-FMAU et un interféron, destiné au traitement ou à la prophylaxie d'un être humain infecté par le virus de l'hépatite B.

8. Utilisation d'un L-FMAU dans la fabrication d'un médicament, lorsqu'il est utilisé en combinaison ou en alternance avec du β-L-FTC et un interféron, destiné au traitement ou à la prophylaxie d'un être humain infecté par le virus de l'hépatite B.

9. Utilisation d'un interféron dans la fabrication d'un médicament, lorsqu'il est utilisé en combinaison ou en alternance avec du β-L-FTC et du L-FMAU, destiné au traitement ou à la prophylaxie d'un être humain infecté par le virus de l'hépatite B.

10. β-L-FTC destiné à être utilisé dans le traitement ou la prophylaxie d'un être humain infecté par le virus de l'hépatite B, dans lequel le β-L-FTC est utilisé en combinaison ou en alternance avec du L-FMAU et un interféron.

11. L-FMAU destiné à être utilisé dans le traitement ou la prophylaxie d'un être humain infecté par le virus de l'hépatite B, dans lequel le L-FMAU est utilisé en combinaison ou en alternance avec du β-L-FTC et un interféron.

12. Interféron destiné à être utilisé dans le traitement ou la prophylaxie d'un être humain infecté par le virus de l'hépatite B, dans lequel l'interféron est utilisé en combinaison ou en alternance avec du β-L-FTC et du L-FMAU.

13. Utilisation selon l'une quelconque des revendications 1 et 7 à 9, dans laquelle l'interféron est libéré sous forme de gène ou de fragment génique, qui exprime la protéine d'interféron.

14. Composition destiné à être utilisée selon ce qui est revendiqué dans la revendication 6, β-L-FTC destiné à être utilisé selon ce qui est revendiqué dans la revendication 10, L-FMAU destiné à être utilisé selon ce qui est revendiqué dans la revendication 11 ou interféron destiné à être utilisé selon ce qui est revendiqué dans la revendication 12, dans lesquels l'interféron se trouve sous forme de gène ou de fragment génique, qui exprime la protéine d'interféron.
